(19) Europäisches Patentamt
European Patent Office
Office européen des brevets

(11) Veröffentlichungsnummer: **0 220 573 B1**

(12) **EUROPÄISCHE PATENTSCHRIFT**

(45) Veröffentlichungstag der Patentschrift: **11.12.91**

(51) Int. Cl.5: **C07D 261/08**, C07D 263/32, C07D 413/04, A61K 31/42

(21) Anmeldenummer: **86114046.5**

(22) Anmeldetag: **10.10.86**

# (54) Heterocyclische Verbindungen.

(30) Priorität: **17.10.85 GB 8525578**
**17.07.86 GB 8617503**

(43) Veröffentlichungstag der Anmeldung:
**06.05.87 Patentblatt 87/19**

(45) Bekanntmachung des Hinweises auf die Patenterteilung:
**11.12.91 Patentblatt 91/50**

(84) Benannte Vertragsstaaten:
**AT BE CH DE FR GB IT LI LU NL SE**

(56) Entgegenhaltungen:
**EP-A- 0 092 239**
**EP-A- 0 111 345**

**CHEMICAL ABSTRACTS, Band 101, nr. 19, 5. November 1984, Seite 693, Zusammenfassung nr. 171150g, Columbus, Ohio, US; D.R.SHRIDHAR et al.: "Potential hypolipidemic agents: part III-synthesis and hypolipidemic activity of 4-(4,5-substituted oxazol-2-yl) phenoxyalkanoic acid derivatives", & Indian J. CHEM., SECT. B 1984, 23B(2), 183-5**

(73) Patentinhaber: **F. HOFFMANN-LA ROCHE AG**
**Postfach 3255**
**CH-4002 Basel(CH)**

(72) Erfinder: **Machin, Peter James**
**42 Parklands Road**
**London(GB)**
Erfinder: **Osbond, John Mervyn**
**4 Lodge Drive**
**Hatfield Herts.(GB)**
Erfinder: **Self, Christopher Raymond**
**56 Bearton Road**
**Hitchin Herts.(GB)**
Erfinder: **Smithen, Carey Ernest**
**17 The Valley Green**
**Welwyn Garden City Herts.(GB)**
Erfinder: **Tong, Brian Peter**
**7 Dalkeith Road**
**Harpenden Herts.(GB)**

(74) Vertreter: **Lederer, Franz, Dr. et al**
**Lederer, Keller & Riederer Patentanwälte**
**Lucile-Grahn-Strasse 22**
**W-8000 München 80(DE)**

**EP 0 220 573 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

PATENTS ABSTRACTS OF JAPAN, Band 10, nr. 71 (C-334)[2128], 20. März 1986; & JP-A-60 20 8971 (HISAMITSU SEIYAKU K.K.) 21-10-1985

## Beschreibung

Die vorliegende Erfindung betrifft heterocyclische Verbindungen der allgemeinen Formel

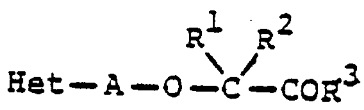

I

worin A $C_{1-6}$-Alkylen,

| | |
|---|---|
| Het | gegebenenfalls heterocyclisch $C_{1-6}$-alkyliertes 2-R-Oxazol-5-yl, 5-R-Oxazol-2-yl, 4-R-Oxazol-2-yl, 2-R-Oxazol-4-yl, 3-R-Isoxazol-5-yl oder 5-R-Isoxazol-3-yl, |
| R | durch Halogen, Trifluormethyl oder $C_{1-6}$-Alkylthio mono- oder disubstituiertes Phenyl oder Thienyl. |
| $R^1$ und $R^2$ | $C_{1-6}$-Alkyl, |
| $R^3$ | Hydroxy, $C_{1-6}$-Alkoxy oder $-NR^4R^5$, |
| $R^4$ und $R^5$ | Wasserstoff oder $c_{1-6}$-Alkyl, oder $-NR^4R^5$ ein Pyrrolidino-, Piperazino-, Morpholino-, oder Thiamorpholino-Ring sind, 3-(4-Chlorphenyl)-5-[[1-methyl-1-(piperidinocarbonyl)äthoxy]-methyl]isoxazol |

und pharmazeutisch verwendbare Salze der Säuren der Formel I, worin $R^3$ Hydroxy ist, mit Basen.

Anstelle der in der Formel I enthaltenen dialkylierten Gruppierung $-OC(R^1,R^2)-$ enthält die Formel I in Patents Abstracts of Japan Vol . 10 No. 71 for JP-A-60 20 8971 die monoalkylierte Gruppierung $-SCH(R^2)-$.

Die Erfindung betrifft ferner die obigen Verbindungen als pharmazeutische, insbesondere gegen Arthritis wirksame Substanzen, ein Verfahren zur Herstellung dieser Verbindungen, pharmazeutische Präparate auf der Basis dieser Verbindungen und die Verwendung dieser Verbindungen zur Herstellung von pharmazeutischen Präparate, insbesondere für die Prophylaxe und Behandlung von Krankheiten, wie Arthritis.

Im Rahmen der Erfindung bezieht sich der Ausdruck $C_{1-6}$ auf geradkettige oder verzweigte Gruppen mit 1-6, vorzugsweise 1-4 C-Atome. Beispiele von $C_{1-6}$-Alkyl, -Alkoxy, -Alkylen bzw. -Alkylthiogruppen sind Methyl, Aethyl, Propyl, Isopropyl, Butyl, s-Butyl, t-Butyl, Pentyl und Hexyl bzw. Methoxy, Aethoxy, Propoxy, Isopropoxy, Butoxy und t-Butoxy bzw. Methylen, 1,1-Aethylen, 1,2-Aethylen, 1,3-Propylen und 1,4-Butylen bzw. Methylthio und Aethylthio. Beispiele von Gruppen $-NR^4R^5$ sind Amino, Methyl- und Aethylamino, Pyrrolidino, Piperazino, Morpholino und Thiamorpholino. Alkylgruppen $R^1$ und $R^2$ können gleich oder verschieden sein. Falls eine Phenyl- oder Thienylgruppe R zwei Substituenten enthält, können diese gleich oder verschieden sein.

Falls $R^1$ und $R^2$ verschieden sind oder A verzweigtes Alkylen ist, enthalten die Verbindungen der Formel I ein asymmetrisches C-Atom und können daher als optisch aktive Verbindungen oder als Racemate vorliegen. Wenn die Verbindungen zwei asymmetrische C-Atome enthalten, können sie als Diastereomere vorliegen. Die Erfindung bezieht sich nicht nur auf die optisch aktiven Enantiomeren sondern auch auf die Racemate und Diastereomere.

In der Formel I ist A vorzugsweise Methylen, 1,1-Aethylen oder 1,2-Aethylen; Het gegebenenfalls durch $C_{1-6}$ -Alkyl, insbesondere Methyl, substituiertes 2-R-Oxazol-5-yl; R Monohalophenyl, insbesondere Monochlorphenyl, wie 4-Chlorphenyl; $R^1$ und $R^2$ Methyl und $R^3$ Hydroxy.

Im Rahmen der Erfindung sind die 2-[[2-(4-Chlorphenyl)-4-methyl -5-oxazolyl]methoxy]-2-methylpropionsäure und das entsprechende Natriumpropionat besonders bevorzugt.

Bevorzugte Verbindungen sind ferner:

2-[[2-(4-Fluorphenyl)-4-methyl-5-oxazolyl]methoxy]-2-methylpropionsäure,
2-[[2-(4-Trifluormethylphenyl)-4-methyl-5-oxazolyl]-methoxy]-2-methylpropionsäure,
2-[[2-(4-Chlorphenyl)-5-oxazolyl]methoxy]-2-methylpropionsäure,
2-[[5-(4-Chlorphenyl)-2-oxazolyl]methoxy]-2-methylpropionsäure,
2-[[4-(4-Chlorphenyl)-2-oxazolyl]methoxy]-2-methylpropionsäure,
2-[[2-(4-Trifluormethylphenyl)-5-methyl-4-oxazolyl]-methoxy]-2-methylpropionsäure,
2-[[2-(4-Chlorphenyl)-5-methyl-4-oxazolyl]methoxy]-2-methylpropionsäure,
2-[[2-(4-Fluorphenyl)-5-methyl-4-oxazolyl]methoxy]-2-methylpropionsäure,
2-[[2-(4-Chlorphenyl)-4-oxazolyl]methoxy]-2-methylpropionsäure,
2-[[2-(3,4-Dichlorphenyl)-4-methyl-5-oxazolyl]methoxy]-2-methylpropionsäure,
2-[[2-(3-Chlorphenyl)-4-methyl-5-oxazolyl]methoxy-2-methylpropionsäure,
2-Methyl-2-[[4-methyl-2-[4-(methylthio)phenyl]-5-oxazolyl]methoxy]propionsäure,

2-[[2-(4-Chlorphenyl)-4-methyl-5-oxazolyl]methoxy]-2-methylpropionamid,
Aethyl 2-[[2-(4-chlorphenyl)-4-methyl-5-oxazolyl]methoxyl]-2-methylpropionat,
2-[[2-(4-Chlorphenyl)-4-äthyl-5-oxazolyl]methoxy]-2-methylpropionsäure,
2-[[2-(4-Chlorphenyl)-4-methyl-5-oxazolyl]methoxy]-2-äthylbuttersäure,
2-[2-[2-(4-Chlorphenyl)-4-methyl-5-oxazolyl]äthoxy]-2-methylpropionsäure,
2-[[2-(4-Chlorphenyl)-4-methyl-5-oxazolyl]methoxy]-2-methylbuttersäure und
Methyl 2-[[2-(4-chlorphenyl)-4-methyl-5-oxazolyl]-methoxy]-2-methylpropionat.

Weitere Beispiele von Verbindungen der Formel I sind:
2-[[3-(4-Chlorphenyl)-5-isoxazolyl]methoxy]-2-methylpropionsäure,
2-[[5-(4-Chlorphenyl)-3-isoxazolyl]methoxy]-2-methylpropionsäure,
Methyl 2-[[3-(4-chlorphenyl)-5-isoxazolyl]methoxy]-2-methylpropionat,
2-[2-[3-(4-Chlorphenyl)-5-isoxazolyl]äthoxy]-2-methylpropionsäure,
Methyl 2-[[3-(3,4-dichlorphenyl)-5-isoxazolyl]methoxy]-2-methylpropionat,
2-[[3-(3,4-Dichlorphenyl)-5-isoxazolyl]methoxy]-2-methylpropionsäure,
2-[1-[3-(4-Chlorphenyl)-5-isoxazolyl]äthoxy]-2-methylpropionsäure,
2-[[3-(5-Chlorthien-2-yl)-5-isoxazolyl]methoxy]-2-methylpropionsäure,
2-[[3-(4-Chlorphenyl)-5-isoxazolyl]methoxy]-2-methylpropionamid,
2-[[3-(4-Chlorphenyl)-5-isoxazolyl]methoxy]-N,2-dimethylpropionamid und
3-(4-Chlorphenyl)-5-[[1-methyl-1-(piperidinocarbonyl)-ethoxy]methyl]isoxazol.

Die erfindungsgemässen Verbindungen können dadurch hergestellt werden, dass man

a) zur Herstellung einer Säure der Formel I, worin $R^3$ Hydroxy ist, einen Alkohol der allgemeinen Formel

Het-A-OH II

mit einem Keton der allgemeinen Formel

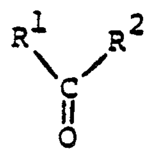

III

worin A, Het, $R^1$ und $R^2$ die im Anspruch 1 angegebene Bedeutung haben,
in Gegenwart eines tri- oder tetrahalogenierten Alkans und einer starken Base umsetzt, oder

b) zur Herstellung einer Verbindung der Formel I, worin $R^3$ $C_{1-6}$-Alkoxy oder $-NR^4R^5$ ist, und $R^4$ und $R^5$ die obige Bedeutung haben, eine Verbindung der allgemeinen Formel

Her-A-X IV

mit einer Verbindung der allgemeinen Formel

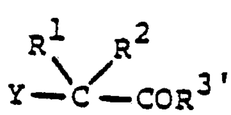

V

worin A, Het, $R^1$ und $R^2$ die obige Bedeutung haben, eines von X und Y Hydroxy und das andere ein Abgangsatom oder eine Abgangsgruppe und $R^{3'}$ $C_{1-6}$-Alkoxy oder $-NR^4R^5$ ist, und $R^4$ und $R^5$ die obige Bedeutung haben,
in Gegenwart einer starken Base umsetzt oder

c) zur Herstellung einer Verbindung der Formel I, worin Het 3-R-Isoxazol-5-yl und $R^3$ $C_{1-6}$-Alkoxy ist, eine Verbindung der allgemeinen Formel

R-C≡N→O VI

mit einer Verbindung der allgemeinen Formel

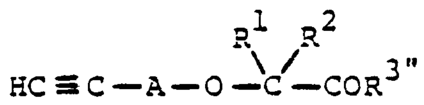

VII

worin R, A, $R^1$ und $R^2$ die im Anspruch 1 angegebene Bedeutung haben und $R^{3''}$ $C_{1-6}$-Alkoxy ist, umsetzt oder

d) zur Herstellung einer Säure der Formel I, worin $R^3$ Hydroxy ist, eine Verbindung der Formel I, worin $R^3$ $C_{1-6}$-Alkoxy ist, hydrolysiert oder

e) zur Herstellung eines Esters der Formel I, worin $R^3$ $C_{1-6}$-Alkoxy ist, eine Verbindung der Formel I, worin $R^3$ Hydroxy ist, verestert oder

f) zur Herstellung eines Amids der Formel I, worin $R^3$ eine Gruppe $-NR^4R^5$ ist, und $R^4$ und $R^5$ die obige Bedeutung haben, eine Verbindung der Formel I, worin $R^3$ Hydroxy oder $C_{1-6}$-Alkoxy ist, amidiert und

g) gewünschtenfalls eine Säure der Formel I, worin $R^3$ Hydroxy ist, in ein pharmazeutisch verwendbares Salz mit einer Base umwandelt.

Beispiele von tri- und tetrahalogenierten Alkanen für die Verfahrensvariante a) sind Bromoform, Jodoform, Tetrachlor- oder Tetrabromkohlenstoff und insbesondere Chloroform. Die starke Base kann z.B. ein Alkalimetallhydroxid, wie Kalium- oder Natriumhydroxid sein. Die Base wird vorzugsweise in fester Form, z.B. als Pulver verwendet. Die Reaktion kann man in einem organischen Lösungsmittel, zweckmässig in überschüssigem Keton der Formel III, bei erhöhter Temperatur, vorzugsweise unter Rückfluss durchführen.

Das Abgangsatom oder die Abgangsgruppe X oder Y in einer Verbindung der Formel IV oder V in der Verfahrensvariante b) kann z.B. ein Halogen wie Chlor oder Brom, ein Alkansulfonat oder ein aromatisches Sulfonat, wie Methansulfonat oder p-Toluolsulfonat sein. Die Reaktion in der Verfahrensvariante b) kann man in Gegenwart eines inerten organischen Lösungsmittels, wie einem aromatischen, gegebenenfalls halogenierten Kohlenwasserstoff, z.B. Benzol, Toluol oder Chlorbenzol, oder vorzugsweise Dimethylformamid (DMF)durchführen. Als starke Base kann man z.B. ein Alkalimetall oder ein Alkalimetallhydrid, wie Natrium oder Kalium, Natriumhydrid oder Kaliumhydrid verwenden. Die Reaktion kann man bei einer Temperatur bis zur Rückflusstemperatur des Reaktionsgemisches, zweckmässig bei Raumtemperatur durchführen.

Die Reaktion in der Verfahrensvariante c) kann man in einem inerten organischen Lösungsmittel, wie einem aliphatischen oder cyclischen Aether, z.B. Diäthyläther, Tetrahydrofuran (THF) oder Dioxan durchführen. Die Reaktion wird zweckmässig bei niedriger Temperatur, z.B. zwischen 0 und 10 °C, vorzugsweise bei etwa 5 °C, durchgeführt.

Die Hydrolyse nach Verfahrensvariante d) kann man in an sich bekannter Weise durchführen, z.B. mit einer Base, wie einem Alkalimetallhydroxid, z.B. Natrium- oder Kaliumhydroxid, in einem Lösungsmittel, wie einem Alkanol, z.B. Methanol oder Aethanol, und bei Raumtemperatur.

Die Veresterung nach der Verfahrensvariante e) kann man in an sich bekannter Weise, z.B. mit einem Diazoalkan, wie Diazomethan, oder mit einem Alkohol, wie Methanol oder Aethanol, in Gegenwart einer starken Säure, wie einer Halogenwasserstoffsäure, z.B. Salzsäure, oder einer Sulfonsäure, z.B. p-Toluolsulfonsäure, durchführen. Man kann auch eine Verbindung der Formel I, worin $R^3$ Hydroxy ist, in an sich bekannter Weise in ein reaktionsfähiges Derivat davon, wie einem Säurehalogenid oder Säureanhydrid, z.B. das Säurechlorrid, umwandeln und letzteres mit einem Alkohol umsetzen.

Die Amidierung nach der Verfahrensvariante f) kann man in an sich bekannter Weise durchführen, z.B. durch Umsetzung einer Verbindung der Formel I, worin $R^3$ $C_{1-6}$-Alkoxy ist, oder eines der oben erwähnten reaktionsfähigen Derivate einer Säure der Formel I, worin $R^3$ Hydroxy ist, mit einer Verbindung der Formel $HNR^4R^5$, z.B. mit Ammoniak, einem Mono- oder Di($C_{1-6}$-alkyl)amin oder einem gesättigten heteromonocyclischen Amin.

Nach der Verfahrensvariante g) kann man die Säuren der Formel I in Salze, z.B. in Alkali- oder Erdalkalimetallsalze, wie Natrium-, Kalium-, Calcium- oder Magnesiumsalze, in Ammoniumsalze oder in Salze mit organischen Aminen, wie Dicyclohexylamin, überführen, z.B. durch Umsetzung der besagten Säure mit einer Base.

Ein Racemat der Formel I kann in die optischen Isomeren aufgetrennt werden. Zum Beispiel kann man ein Racemat der Formel I, worin $R^3$ Hydroxy ist, mit einer optisch aktiven Base wie (+)- oder (-)-Ephedrin, (+)- oder (-)-$\alpha$-Methylbenzylamin, behandeln, die optisch aktiven Salze, z.B. durch fraktionierte Kristallisation trennen und gewünschtenfalls die optisch einheitlichen Verbindungen in an sich bekannter Weise aus diesen Salzen isolieren. Alternativ kann man optisch aktive Ausgangsmaterialien einsetzen.

Die Ausgangsmaterialien sind bekannt oder Analoga von bekannten Verbindungen und können in

Analogie zu letzteren, wie in den Beispielen beschrieben, hergestellt werden.

Die Verbinungen der Formel I und die pharmazeutisch verwendbaren Salze der Säuren der Formel I, worin $R^3$ Hydroxy ist, mit Basen haben antiarthritische Wirksamkeit, was wie folgt demonstriert werden kann:

Weiblichen Ratten, bei denen eine Pfotenentzündung erzeugt worden war, wurde die Testverbindung während 15 Tagen oral verabreicht. Die Aktivität ist in der nachfolgenden Tabelle als mittlerer Unterschied zwischen den Pfotenvolumina während und vor der Verabreichung der Testverbindung angegeben.

Tabelle

| Produkt des Beispiels Nr. | Dosis in mg/kg/Tag | Mittlerer Unterschied zwischen den Pfotenvolumina am 11. und am 1. Tag (ml) |
|---|---|---|
| 1 | 50 | -0.29 ± 0.05 |
| 8 | 100 | -0.44 ± 0.11 |
| 10 | 100 | -0.47 ± 0.06 |
| Kontrolle | - | +0.13 ± 0.12 |

Die Verbindungen der Formel I und die pharmazeutisch verwendbaren Salze der Säuren der Formel I, worin $R^3$ Hydroxy ist, mit Basen können als Medikamente, z.B. in Form pharmazeutischer Präparate Verwendung finden. Man kann diese oral, z.B. in Form von Tabletten, Dragées, Hart- oder Weichkapseln, Lösungen, Emulsionen oder Suspensionen verabreichen. Die Verabreichung kann auch rektal, z.B. in Form von Suppositorien, oder parenteral, z.B. in Form von Injektionen durchgeführt werden.

Solche Medikamente können in an sich bekannter Weise hergestellt werden, in dem man eine oder mehrere der erfindungsgemässen Verbindungen und gegebenenfalls ein oder mehrere weitere therapeutisch aktive Verbindungen zusammen mit einem oder mehreren therapeutisch inerten Excipientien in eine galenische Verabreichungsform bringt. Beispiele von inerten anorganischen oder organischen Trägern für Tabletten, Dragées und Hartgelatinekapseln sind Laktose, Maisstärke oder Derivate davon, Talk, Stearinsäure oder ihre Salze. Beispiele von Trägern für Weichgelatinekapseln sind pflanzliche Oele, Wachse, Fette, halbfeste oder flüssige Polyole. Je nach der Natur der Aktivsubstanz erübrigt sich jedoch der Träger bei Weichgelatinekapseln. Beispiele von Trägern für die Herstellung von Lösungen und Sirupen sind Wasser, Polyole, Saccharose, Invertzucker oder Glukose. Beispiele von Trägern für Injektionslösungen sind Wasser, Alkohole, Polyole, Glyzerin oder pflanzliche Oele. Beispiele von Trägern für Suppositorien sind in der Natur vorkommende oder behandelte Oele, Wachse, Fette, halbflüssige oder flüssige Polyole. Die pharmazeutischen Präparate können daneben noch Konservierungsmittel, Lösungsvermittler, Stabilisierungsmittel, Netzmittel, Emulgiermittel, Süssmittel, Färbemittel, Aromatisierungsmittel, Salze zur Veränderung des osmotischen Drukes, Puffer, Ueberzugsmittel oder Antioxidantien enthalten. Die Dosierung kann innerhalb weiter Grenzen variieren und ist natürlich in jedem einzelnen Fall den individuellen Gegebenheiten anzupassen. Bei oraler Verabreichung beträgt die Tagesdosis etwa 50 bis 2500 mg.

Beispiel 1

6,9 g (30,8 mMol) 2-(4-Chlorphenyl)-4-methyl-5-oxazolmethanol wurden mit 6,2 g (155 mMol) pulverförmiges Natriumhydroxid in 45 ml Aceton vermischt und die Suspension wurde unter Rückfluss erhitzt. Dann wurden 4,9 g (41,1 mMol) Chloroform in 10 ml Aceton zugetropft und die Suspension wurde 4 Stunden bei Rückflusstemperatur erhitzt. Das Lösungsmittel wurde abgedampft und der Rückstand zwischen 150 ml Diäthyläther und 400 ml Wasser verteilt. Die organische Phase wurde abgetrennt und die wässrige Phase mit Diäthyläther extrahiert. Die wässrige Phase wurde auf pH 2 mit konzentrierter Salzsäure angesäuert und dreimal mit 50 ml Dichlormethan extrahiert. Die vereinigten Dichlormethanextrakte wurden getrocknet, filtriert und eingedampft. Man erhielt 3,7 g rohes Produkt und nach Umkristallisieren aus Aethylacetat 3,1 g (32,5%) 2-[[2-(4-Chlorphenyl)-4-methyl -5-oxazolyl]methoxy]-2-methylpropionsäure, Smp. 166-167° C.

Das Ausgangsmaterial wurde wie folgt hergestellt:

a) Eine Lösung von 15,65 g 4-Chlorbenzoesäure in 20 ml DMF wurde unter Rühren einer Suspension von 5,3 g wasserfreiem Natriumcarbonat in 80 ml DMF zugetropft. Die Suspension wurde eine Stunde bei 80° C erhitzt und eine Lösung von 16,46 g Aethyl-2-chloracetoacetat in 10 ml DMF wurde zugesetzt. Die Suspension wurde 6 Stunden bei 80° C erhitzt und dann über Nacht abkühlen gelassen. Das Lösungsmittel wurde abgedampft und der Rückstand zwischen 400 ml Wasser und 300 ml Diäthyläther

verteilt. Die organische Phase wurde abgetrennt und zweimal mit 300 ml Wasser gewaschen. Die organische Phase wurde getrocknet und filtriert und das Lösungsmittel wurde abgedampft, wobei man 26,86 g (94%) Aethyl 2-(4-chlorbenzoyloxy)-3-oxobutyrat erhielt.

b) 19,6 g konzentrierte Schwefelsäure wurde zu 90 g bei 10° C gehaltenem Formamid getropft. 26,8 g des Produktes von a) wurden zugesetzt und die Lösung wurde unter Rühren 2 Stunden auf 140° C erhitzt. Die abgekühlte Suspension wurde zwischen 400 ml Wasser und 300 ml Diäthyläther verteilt. Die organische Phase wurde zweimal mit 200 ml 1N Salzsäure und einmal mit 200 ml Wasser gewaschen und dann filtriert. Die organische Phase wurde getrocknet, filtriert und eingedampft. Der Rückstand wurde auf Silicagel mit 20 Vol.% Aethylacetat in Hexan chromatographiert. Nach Abdampfen des Eluats erhielt man 13,3 g (50%) Aethyl 2-(4-chlorphenyl)-4-methyl-5-oxazolcarboxylat, Smp. 80-180° C.

c) 9,3 g des Produktes von b) wurden in 40 ml trockenem THF gelöst und die Lösung wurde zu einer Suspension von 1,75 g $LiAlH_4$ in 60 ml trockenem THF bei 0° C unter Rühren getropft. Die Suspension wurde eine Stunde bei 0° C und 1,5 Stunden bei Raumtemperatur gerührt. Ueberschüssiges $LiAlH_4$ wurde durch Zusatz einer gesättigten Natriumsulfatlösung, gefolgt von 100 ml verdünnter Schwefelsäure zerstört. Die wässrige Phase wurde zweimal mit Dichlormethan extrahiert. Die vereinigten Extrakte wurden mit Wasser gewaschen, getrocknet, filtriert und eingedampft. Der Rückstand wurde aus Chlorform/Hexan umkristallisiert und man erhielt 6,44 g (82%) 2-(4-Chlorphenyl)-4-methyl-5-oxazolmethanol, Smp. 135° C.

Beispiel 2

Analog Beispiel 1 erhielt man aus 8,5 g (41,1 mMol) 2-(4-Fluorphenyl)-4-methyl-5-oxazolmethanol, Smp. 136° C, nach Umkristallisieren aus Aethylacetat 3,5 g (29%) 2-[[2-(4-Fluorphenyl)-4-methyl-5-oxazoly]-methoxy]-2-methylpropionsäure, Smp. 166,5-167,5° C.

Beispiel 3

Analog Beispiel 1 erhielt man aus 13,5 g (57,9 mMol) 2-(4-Trifluormethylphenyl)-4-methyl-5-oxazolmethanol, Smp. 141-142° C, nach Umkristallisieren aus Aethylacetat 5,67 g (28,5%) 2-[[2-(4-Trifluormethylphenyl)-4-methyl -5-oxazolyl]methoxy]-2-methylpropionsäure, Smp. 156-157° C.

Beispiel 4

Analog Beispiel 1 erhielt man aus 4, 25 g (20,3 mMol) 2-(4-Chlorphenyl)-5-oxazolmethanol nach Umkristallisieren aus Aethylacetat 2,2 g (36,6%) 2-[[2-(4-Chlorphenyl)-5-oxazolyl]methoxy] -2-methylpropionsäure, Smp. 151,5-153° C.

Das Ausgangsmaterial wurde wie folgt hergestellt:

13,8 ml einer 2,42M Lösung von n-Butyllithium in Hexan wurden zu einer Lösung von 6 g 2-(4-Chlorphenyl)oxazol in 60 ml trockenem THF bei -60° C unter Argon getropft. Die Suspension wurde auf -40° C erwärmt und dann wurden 6,9 g Paraformaldehyd in 50 ml trockenem THF zugesetzt. Das Gemisch wurde auf Raumtemperatur erwärmt und eine Stunde gerührt. Dem Gemisch wurden 10 ml gesättigte Ammoniumchloridlösung zugesetzt und die entstandene Suspension wurde zwischen 300 ml Wasser und 200 ml Dichlormethan verteilt. Die organische Phase wurde abgetrennt und die wässrige Phase zweimal mit 200 ml Dichlormethan extrahiert. Die vereinigten organischen Phasen wurden getrocknet und filtriert und das Lösungsmittel wurde abgedampft. Der Rückstand wurde auf Silicagel mit 70 Vol.% Aethylacetat in Hexan chromatographiert. Das Eluat wurde zu 4,25 g 2-(4-Chlorphenyl)-5-oxazolmethanol, Smp. 115-116° C eingedampft.

Beispiel 5

Analog Beispiel 1 erhielt man aus 7,2 g (34,4 mMol) 5-(4-Chlorphenyl)-2-oxazolmethanol, nach Umkristallisieren aus Aethylacetat 1,4 g (14%) 2-[[5-(4-Chlorphenyl) -2-oxazolyl]methoxy]-2-methylpropionsäure, Smp. 143-146° C. Das Ausgangsmaterial wurde wie folgt hergestellt:

a) 12,78 g N-[(4-Chlorbenzoyl)methyl]-2-äthoxyacetamid wurden in 32,5 ml konzentrierter Schwefelsäure unter Rühren und Abkühlen gelöst. Das Gemisch wurde 20 Stunden bei Raumtemperatur gehalten, dann auf 600 ml Eiswasser geschüttet und dreimal mit 150 ml Diäthyläther extrahiert. Die vereinigten Aetherextrakte wurden in 100 ml gesättigter Natriumbicarbonatlösung gewaschen, dann getrocknet, filtriert und zu 10,55 g (89%) 5-(4-Chlorphenyl)-2-äthoxymethyloxazol eingedampft.

b) 7,5 ml konzentrierte Schwefelsäure wurden einer Suspension von 1,4 g des Produktes von a) in 4,5 ml Wasser unter Abkühlen zugesetzt. Die entstandene Lösung wurde 4 Stunden unter Rühren bei 140 °C erhitzt, dann abgekühlt und auf 140 ml Eiswasser geschüttet. Das Gemisch wurde dreimal mit 30 ml Dichlormethan extrahiert. Die vereinigten organischen Extrakte wurden mit 50 ml gesättigter Natriumbicarbonatlösung gewaschen, getrocknet, filtriert und eingedampft. Nach Umkristallisieren aus Toluol erhielt man 0,8 g (64%) 5-(4-Chlorphenyl)-2-oxazolmethanol, Smp. 100-103,5 °C.

Beispiel 6

Analog Beispiel 1 erhielt man aus 16 g (76 mMol) 4-(4-Chlorphenyl)-2-oxazolmethanol nach Umkristallisieren aus Toluol, 8,85 g (39%) 2-[[4-(4-Chlorphenyl)-2-oxazolyl]-methoxy] -2-methylpropionsäure, Smp. 115,5-118 °C.

Das Ausgangsmaterial wurde wie folgt hergestellt:

a) 40 g konzentrierte Schwefelsäure wurden unter Rühren zu 150 g bei 10 °C gehaltenem Formamid getropft. Nach Zusatz von 50 g (4-Chlorphenyl)methyläthoxyacetat wurde die Lösung 2 Stunden unter Rühren bei 140 °C erhitzt. Das Gemisch wurde abgekühlt, auf 2 l Wasser gegossen durch Zusatz von konzentrierter Salzsäure auf pH 1 gestellt und einmal mit 500 ml und zweimal mit 250 ml Toluol extrahiert. Die vereinigten Toluolextrakte wurden mit Wasser gewaschen, getrocknet, filtriert und eingedampft. Der Rückstand wurde mit 700 ml heissem Petroläther (s.p. 60-80 °C) extrahiert. Die Lösung wurde zur Trockene eingedampft und der Rückstand in 150 ml heissem Petroläther (s.p. 40-60 °C) wieder gelöst. Nach Kühlung in Aceton/$CO_2$ wurde das Produkt abfiltriert und getrocknet. Man erhielt 21,7 g (47%) 4-(4-Chlorphenyl)-2-äthoxymethyloxazol.

b) 181 ml einer 1M Lösung von Bortrichlorid in Dichlormethan wurden unter Rühren zu einer Lösung von 21,5 g 4-(4-Chlorphenyl)-2-äthoxymethyloxazol in 100 ml Dichlormethan getropft. Die Temperatur des Gemisches wurde unterhalb von 10 °C gehalten. Danach wurde das Gemisch 2 Stunden bei Raumtemperatur gerührt und dann auf 500 ml Eiswasser gegossen. Die organische Phase wurde abgetrennt und die wässrige Phase mit 100 ml Dichlormethan extrahiert. Die vereinigten organischen Extrakte wurden mit gesättigter Natriumbicarbonatlösung gewaschen, getrocknet, filtriert und eingedampft. Nach Umkristallisieren des Rückstandes aus Toluol erhielt man 15,7 g (83%) 4-(4-Chlorphenyl)-2-oxazolmethanol, Smp. 68-170,5 °C.

Beispiel 7

Analog Beispiel 1 erhielt man aus 23,15 g (90 mMol) 2-(4-Trifluormethylphenyl)-5-methyl-4-oxazolmethanol nach Umkristallisieren aus Toluol 8,0 g (26%) 2-[[2-(4-Trifluormethylphenyl)-5-methyl -4-oxazolyl]-methoxy]-2-methylpropionsäure, Smp. 146-147 °C.

Das Ausgangsmaterial wurde wie folgt hergestellt:

a) 34,8 g 4-Trifluormethylbenzaldehyd und 20,2 g 2,3-Butandionmonoxim wurden in 100 ml Eisessig gelöst und das Gemisch wurde unter Rühren bei Raumtemperatur mit Chlorwasserstoff gesättigt. Das Gemisch wurde über Nacht bei Raumtemperatur gerührt und dann mit einem Ueberschuss an wasserfreiem Diäthyläther behandelt. Der Niederschlag wurde abfiltriert, mit wasserfreiem Diäthyläther gewaschen und getrocknet. Man erhielt 50 g (86%) 2-(4-Trifluormethylphenyl)-4,5-dimethyloxazol -N-oxid-hydrochlorid, Smp. 175-177 °C (Zersetzung).

b) 50 g des Produktes von a) wurden einer Suspension von 14 g wasserfreiem Natriumacetat in 100 ml Essigsäureanhydrid zugesetzt, das Gemisch wurde gerührt und dann 4 Stunden bei 140 °C erhitzt. Das Gemisch wurde abgekühlt und der Niederschlag abfiltriert und mit Toluol gewaschen. Die vereinigten organischen Lösungen wurden konzentriert und der Rückstand wurde zwischen Dichlormethan und überschüssigem 2N Natriumcarbonat verteilt. Die organische Phase wurde abgetrennt, zweimal mit Wasser gewaschen, getrocknet, durch Silicagel filtriert und konzentriert. Man erhielt 30 g (60%) 4-Acetoxymethyl-2-(4-trifluormethylphenyl)-5-methyloxazol.

c) 30 g des obigen Esters wurden in 400 ml Aethanol gelöst und die Lösung wurde mit 300 ml 2N Natriumhydroxid behandelt. Das Gemisch wurde über Nacht bei Raumtemperatur gerührt, dann konzentriert und der Rückstand zwischen Dichlormethan und Wasser verteilt. Die wässrige Phase wurde abgetrennt und zweimal mit Dichlormethan gewaschen. Die vereinigten organischen Phasen wurden mit Wasser gewaschen, getrocknet, filtriert und konzentriert. Man erhielt 23 g (90%) rohes 2-(4-Trifluormethylphenyl)-5-methyl-4-oxazolmethanol, das nach Umkristallisieren aus Cyclohexan in Gegenwart von Aktivkohle bei 98-99 °C schmolz.

Beispiel 8

Analog Beispiel 1 erhielt man aus 11,2 g (50 mMol) 2-(4-Chlorphenyl)-5-methyl-4-oxazolmethanol, Smp. 130-131 °C, nach Umkristallisieren aus Toluol 2,7 g (17,4%) 2-[[2-(4-Chlorphenyl)-5-methyl-4-oxazolyl]-methoxy] -2-methylpropionsäure, Smp. 123-124 °C.

Das Ausgangsmaterial wurde entweder analog Beispiel 1c) aus 2-(4-Chlorphenyl)-5-methyl-4-oxazolcarbonsäure oder analog Beispiel 7b) und c) aus 2-(4-Chlorphenyl)-4,5-dimethyloxazol-N-oxid hergestellt.

Beispiel 9

Analog Beispiel 1 erhielt man aus 6,5 g (31,4 mMol) 2-(4-Fluorphenyl)-5-methyl-4-oxazolmethanol nach Umkristallisieren aus Toluol 1,5 g (17%) 2-[[2-(4-Fluorphenyl)-5-methyl-4-oxazolyl]methoxy] -2-methylpropionsäure, Smp. 118-120 °C.

Das Ausgangsmaterial, Smp. 153-155 °C, wurde analog Beispiel 8 ausgehend von 2-(4-Fluorphenyl)-5-methyl-4-oxazolcarbonsäure oder durch Umwandlung des 2-(4-Fluorphenyl)-4,5-dimethyloxazol-N-oxids in Essigsäureanhydrid gefolgt von wässrig-alkoholischer alkalischer Hydrolyse.

Beispiel 10

Eine Lösung von 1,55 g (13,2 mMol) Methyl-2-hydroxy-2-methylpropionat in 4 ml DMF wurde unter Rühren einer Suspension von 0,53 g (13,2 mMol) zu einer Dispersion von 60% NaH in Mineralöl in 8 ml trockenem DMF getropft. Die Lösung wurde eine Stunde bei Raumtemperatur gerührt und dann einer Lösung von 2 g (9,4 mMol) 2-(4-Chlorphenyl)-4-chlormethyloxazol (das wie beschrieben im britischen Patent Nr. 1,139,940 hergestellt wurde) in 4 ml trockenem DMF bei 0 °C unter Argon zugesetzt. Das Gemisch wurde über Nacht bei Raumtemperatur gerührt und dann wurden 2 ml gesättigte Ammoniumchloridlösung zugesetzt. Der grösste Teil des Lösungsmittels wurde abgedampft und der Rückstand zwischen 100 ml Diäthyläther und 100 ml Wasser verteilt. Die organische Phase wurde abgetrennt, mit 100 ml Wasser gewaschen und getrocknet. Die organische Lösung wurde filtriert und eingedampft. Man erhielt, 2,7 g Feststoff, das in 100 ml Aethanol gelöst und und mit 0,4 g (10 mMol) Natriumhydroxid in 5 ml Wasser versetzt wurde. Die Lösung wurde unter Rückfluss erhitzt, dann abgekühlt und der grösste Teil des Lösungsmittels wurde abgedampft. Der feste Rückstand wurde in 400 ml Wasser gelöst und zweimal mit 100 ml Diäthyläther extrahiert. Die wässrige Phase wurde mit konzentrierter Salzsäure auf pH 2 angesäuert und dreimal mit 100 ml Dichlormethan extrahiert. Die vereinigten organischen Extrakte wurden getrocknet, filtriert und zu einem Feststoff eingedampft. Dieses wurde aus Aethylacetat umkristallisiert und man erhielt 1,6 g (57%) 2-[[2-(4-Chlorphenyl)-4-oxazolyl]methoxy] -2-methylpropionsäure, Smp. 144,5-145 °C.

Beispiel 11

3,57 g (37 mMol) Triäthylamin in 30 ml trockenem DMF wurden unter Rühren und Abkühlen auf 10 °C zu einer Lösung von 6,33 g (33,3 mMol) 4-Chlorbenzol-N-hydroxycarboximidoylchlorid in 60 ml trockenem DMF getropft. Nach einer Stunde bei 20 °C wurde die erhaltene Lösung von 4-Chlorbenzonitriloxid in Wasser gegossen und abfiltriert. Der feuchte Feststoff wurde in 150 ml Diäthyläther gelöst und zweimal mit 10 g Molekularsieb getrocknet. Die ätherische Lösung wurde filtriert und bei 5 °C mit 6,56 g (36,6 mMol) Methyl-2-propynyloxy-2-methylpropionat in 25 ml Diäthyläther versetzt. Nach 24 Stunden wurde der Diäthyläther entfernt und das Produkt wurde auf 220 g Silicagel mit Hexan/Aethylacetat (4:1) und dann Hexan/Aethylacetat (2:1) chromatographiert. Man erhielt 4,67 g Methyl 2-[[3-(4-chlorphenyl)-5-isoxazolyl]-methoxy]-2-methylpropionat in Form eines farblosen Oels von 99% Reinheit.

Das Ausgangsmethylpropionat wurde wie folgt hergestellt:

55 g Methyl 2-hydroxy-2-methylpropionat in 400 ml trockenem DMF wurden unter Rühren zu 14 g einer Dispersion von Natriumhydrid in 200 ml trockenem DMF getropft. Nach 3 Stunden bei 20 °C wurden 62,5 g Propargylbromid (in Form einer 80%-igen Toluollösung) in 200 ml DMF unter Rühren unterhalb von 25 °C zugesetzt. Nach 17-stündigem Rühren wurde das Gemisch auf Eis gegossen und mit Toluol extrahiert. Die Toluolphase wurde eingedampft und der Rückstand bei 80 °C/15 mmHg destilliert. Man erhielt 26,8 g (41%) Methyl-2-propynyloxy-2-methylpropionat.

Beispiel 12

1,3 ml 1N Natriumhydroxid wurden 0,31 g (1,0 mMol) Methyl-2-[[3-(4-chlorphenyl)-5-isoxazolyl]-

methoxy]-2-methylpropionat in 10 ml Methanol zugesetzt. Nach 64 Stunden bei 20° C wurde das Methanol abgedampft und das Produkt durch Ansäuern mit 2N Salzsäure und Extraktion mit Diäthyläther isoliert. Nach Kristallisieren aus Toluol/Hexan erhielt man 0,17 g (58%) 2-[[3-(4-Chlorphenyl)-5-isoxazolyl]methoxy] -2-methylpropionsäure, Smp. 130-134° C.

Beispiel 13

Analog Beispiel 1 erhielt man aus 5 g (24 mMol) 5-(4-Chlorphenyl)-3-isoxazolmethanol nach Umkristallisieren aus Toluol/Hexan 1,06 g (15%) 2-[[5-(4-Chlorphenyl)-3-isoxazolyl]methoxy] -2-methylpropionsäure, Smp. 115-118° C.

Das Ausgangsmaterial wurde wie folgt hergestellt:

a) 4,75 g Aethyl-5-(4-chlorphenyl)-3-isoxazolcarboxylat, Smp. 124-126° C (hergestellt wie beschrieben in J. Het. Chem., 19, 1982, 557) wurden in 30 ml Diäthyläther unter einer Stickstoffatmosphäre gelöst. Eine Aufschlämmung von 1,05 g Lithiumborhydrid in 5 ml Diäthyläther wurde zugetropft. Das Gemisch wurde 15 Minuten bei 20° C gerührt und dann 3,5 Stunden unter Rückfluss erhitzt. Das Gemisch wurde dann in Eiswasser gegossen und mit Diäthyläther extrahiert. Die vereinigten Aetherextrakte wurden getrocknet, filtriert und eingedampft. Nach Umkristallisieren des Rückstandes aus Toluol erhielt man 2,8 g (71%) 5-(4-Chlorphenyl)-3-isoxazolmethanol, Smp. 93-95° C.

Beispiel 14

Analog Beispiel 1 erhielt man aus 5 g (22,4 mMol) 3-(4-Chlorphenyl)-5-isoxazolyl-2-äthanol nach Umkristallisieren aus Aethylacetat/Hexan 0,9 g (13%) 2-[2-[3-(4-Chlorphenyl)-5-isoxazolyl]äthoxy] -2-methylpropionsäure, Smp. 98-100° C.

Das Ausgangsmaterial wurde wie folgt hergestellt:

5,05 g Triäthylamin in 20 ml Diäthyläther wurden bei -10° C zu einer Lösung von 3,86 g 3-Butyn-1-ol und 9,50 g 4-Chlorbenzol-N-hydroxycarboximidoylchlorid in 200 ml trockenem Diäthylather getropft. Nach 3 Stunden wurde das Reaktionsgemisch filtriert, das Filtrat wurde eingedampft und der Rückstand aus Aethylacetat/Hexan umkristallisiert. Man erhielt 6,15 g (55%) 3-(4-Chlorphenyl)-5-isoxazolyl-2-äthanol, Smp. 65,5-67,5° C nach Kristallisieren aus Aethylacetat.

Beispiel 15

2,02 g (0,02 Mol) Triäthylamin in 20 ml Diäthyläther wurden bei -10° C zu einer Lösung von 4,49 g (0,02 Mol) 3,4-Dichlorbenzol-N-hydroxycarboximidoylchlorid in 90 ml trockenem Diäthyläther getropft. Nach 0,5 Stunden bei -5 bis -10° C wurden 4,004 g (0,022 Mol) Methyl-2-propynyloxy-2-methylpropionat zugesetzt und das Gemisch wurde 48 Stunden bei -5° C gehalten. Das Gemisch wurde filtriert, das Filtrat eingedampft und der Rückstand auf 150 g Silicagel mit Hexan/Aethylacetat (4:1) chromatographiert. Man erhielt 6 g (87%) reines Methyl-2-[[3-(3,4-dichlorphenyl)-5-isoxazolyl]methoxy] -2-methylpropionat in Form eines Oels.

Mikroanalyse für $C_{15}H_{15}Cl_2NO_4$:

Berechnet: C: 52,34; H: 4,39; N: 4,07%
Gefunden: C: 52,57; H: 4,38; N: 4,15%.

Beispiel 16

Analog Beispiel 12 erhielt man aus 1,88 g (5 mMol) Methyl-2-[[2-(3,4-dichlorphenyl) -5-isoxazolyl]-methoxy]-2-methylpropionat nach Umkristallisieren aus Aethylacetat 1,11 g (69%) 2-[[3-(3,4-Dichlorphenyl)-5-isoxazolyl]methoxy]-2-methylpropionsäure, Smp. 129-131° C.

Beispiel 17

Analog Beispiel 1 erhielt man aus 5 g (22,4 mMol) 3-(4-Chlorphenyl)-5-isoxazolyl-1-äthanol nach Umkristallisieren aus Aethylacetat/Hexan 0,7 g (10%) 2-[1(RS)-[3-(4-Chlorphenyl)-5-isoxazolyl]äthoxy] -2-methylpropionsäure, Smp. 141-143° C.

Diese (RS)-Säure wurde wie folgt aufgetrennt:

Ein Gemisch von 6,18 g (200 mMol) der (RS)-Säure und 3,32 g (200 mMol) (+)-Ephedrin wurde aus 20 ml Isopropanol umkristallisiert. Die Kristalle wurden sechsmal aus Isopropanol umkristallisiert. Man erhielt

reines (-)-Ephedrinsäuresalz. Dieses wurde zwischen 2N Salzsäure und Diäthyläther verteilt. Der Aetherextrakt wurde getrocknet, filtriert und eingedampft. Nach Umkristallisieren aus Aethylacetat/Hexan erhielt man (-)-2-[1-[3-(4-Chlorphenyl)-5-isoxazolyl]äthoxy] -2-methylpropionsäure, Smp. 121-125° C, $[\alpha]_{589}$ = -96,1° (c = 1% in Aethanol).

Analog, jedoch unter Verwendung von (-)-Ephedrin erhielt man (+)-2-[1-[3-(4-Chlorphenyl)-5-isoxazolyl]-äthoxy]-2-methylpropionsäure, Smp. 120-124° C, $[\alpha]_{589}$ = +94.7° (c = 1% in Aethanol).

Das Ausgangsmaterial wurde analog Beispiel 14 aus 1,93 g 3-Butyn-2-ol hergestellt. Nach Umkristallisieren aus Methylcyclohexan erhielt man 3,82 g (68%) 3-(4-Chlorphenyl)-5-isoxazolyl-1-äthanol, Smp. 63-65° C.

Beispiel 18

Ein Gemisch von 4,9 g (25 mMol) 5-Chlorthiophen-N-hydroxycarboximidoylchlorid und 4,54 g (27 mMol) Methyl-2-propynyloxy-2-methylpropionat in 180 ml trockenem Diäthyläther wurde mit einer Lösung von 2,53 g (25 mMol) Triäthylamin in 20 ml trockenem Diäthyläther unter Stickstoff unter Rühren behandelt. Nach 3 Tagen bei 20° C wurde das Gemisch filtriert und die Filtrate wurden eingedampft. Man erhielt 6,6 g rohes Methyl-2-[[3-(5-chlorthien-2-yl) -5-isoxazolyl]methoxy]-2-methylpropionat, wovon 6,3 g wie im Beispiel 12 beschrieben hydrolysiert wurden. Man erhielt nach Kristallisieren aus Aethylacetat/Hexan 2,1 g (28%) 2-[[3-(5-Chlorthien-2-yl) -5-isoxazolyl]methoxy]-2-methylpropionsäure, Smp. 128-130° C.

Beispiel 19

1,3 g Oxalylchlorid wurden zu 3 g (100 mMol) 2-[[3-(4-Chlorphenyl)-5-isoxazolyl]methoxy] -2-methylpropionsäure in 100 ml Diäthyläther getropft. Nach 4 Stunden bei 20° C wurde der Diäthyläther abgedampft, das Säurechlorid in 75 ml THF aufgenommen und einer mit Ammoniakgas gesättigten THF-Lösung zugesetzt. Nach 1,5 Stunden wurde der Niederschlag abfiltriert, die Filtrate wurden eingedampft und die erhaltenen 2,46 g (82%) 2-[[3-(4-Chlorphenyl)-5-isoxazolyl]methoxy]-2-methylpropionamid aus Aethanol kristallisiert, Smp. 154-155° C.

Beispiel 20

Analog Beispiel 19 erhielt man unter Verwendung von Methylamin in trockenem Diäthyläther 2-[[3-(4-Chlorphenyl)-5-isoxazolyl]methoxy] -N,2-dimethylpropionamid, Smp. 93-94° C nach Kristallisieren aus Aethylacetat.

Beispiel 21

Analog Beispiel 19 erhielt man unter Verwendung von Piperidin in trockenem Diäthyläther 3-(4-Chlorphenyl)-5-[[1-methyl-1-(piperidinocarbonyl) äthoxy]methyl]isoxazol, Smp. 239-242° C nach Kristallisieren aus Aethylacetat/Hexan.

Beispiel 22

Analog Beispiel 1 erhielt man aus 5,17 g (20 mMol) 2-(3,4-Dichlorphenyl)-4-methyl -5-oxazolmethanol, Smp. 158-159° C, nach Umkristallisieren aus Aethylacetat/Hexan 1,69 g (24,6%) 2-[[2-(3,4-Dichlorphenyl)-4-methyl -5-oxazolyl]methoxy]-2-methylpropionsäure, Smp. 130-131° C.

Beispiel 23

Analog Beispiel 1 erhielt man aus 4,6 g (20,6 mMol) 2-(3-Chlorphenyl)-4-methyl-5-oxazolmethanol, Smp. 123° C, nach Umkristallisieren aus Aethylacetat/Hexan 1,2 g (18,8%) 2-[[2-(3-Chlorphenyl)-4-methyl -5-oxazolyl]methoxy]-2-methylpropionsäure, Smp. 117° C.

Beispiel 24

Analog Beispiel 1 erhielt man aus 5 g (21,3 mMol) 4-Methyl-2-[4-(methylthio)phenyl]-5-oxazolmethanol, Smp. 130° C, nach Umkristallisieren aus Aethylacetat/Hexan 0,4 g (6%) 2-Methyl-2-[[4-methyl-2-[4-(methylthio)phenyl] -5-oxazolyl]methoxy]propionsäure, Smp. 128° C.

Beispiel 25

Einer Lösung von 4,44 g (14,5 mMol) 2-[[2-(4-Chlorphenyl) -4-methyl-5-oxazolyl]methoxy]-2-methylpropionsäure in 40 ml Dichlormethan wurden ein Tropfen DMF und 1,96 g (16 mMol) Thionylchlorid zugesetzt. Das Gemisch wurde 4 Stunden unter Rückfluss erhitzt. Die abgekühlte Lösung des entstandenen Säurechlorids wurde unter Rühren 70 ml einer Ammoniumhydroxidlösung (spezifisches Gewicht 1,880) und Eis zugesetzt. Nach 16 Stunden Rühren wurde die Suspension mit 200 ml Dichlormethan extrahiert. Die organische Phase wurde abgetrennt und zweimal mit 100 ml Wasser gewaschen. Die organische Phase wurde getrocknet und filtriert und das Lösungsmittel abgedampft. Nach Umkristallisieren aus Aethylacetat erhielt man 2,2 g (49%) 2-[[2-(4-Chlorphenyl)-4-methyl-5-oxazolyl]methoxy] -2-methylpropionamid, Smp. 177-178° C.

Beispiel 26

Einer Lösung von 4,44 g (14,5 mMol) 2-[[2-(4-Chlorphenyl)-4-methyl-5-oxazolyl]methoxy] -2-methylpropionsäure in 40 ml trockenem Dichlormethan wurden ein Tropfen DMF und 1,9 g (16 mMol) Thionylchlorid zugesetzt. Das Gemisch wurde 4 Stunden unter Rückfluss erhitzt. 70 ml Aethanol wurden unter Rühren der erhaltenen Säurechloridlösung bei 0° C zugesetzt und das Gemisch wurde 18 Stunden gerührt. Das Lösungsmittel wurde abgedampft und der Rückstand auf Silicagel mit 17 Vol.% Aethylacetat in Hexan chromatographiert. Nach Abdampfen des Eluats erhielt man 2,8 g (57,2%) Aethyl 2-[[2-(4-chlorphenyl)-4-methyl-5-oxazolyl]methoxy]-2-methylpropionat, Smp. 75-76° C.

Beispiel 27

Analog Beispiel 1 erhielt man aus 2 g (8,4 mMol) 2-(4-Chlorphenyl)-4-äthyl-5-oxazolmethanol, Smp. 135-136° C (hergestellt aus 4-Chlorbenzoesäure und Aethyl-2-chlor-3-oxopentanoat), nach Umkristallisieren aus Aethylacetat/Hexan 1,09 g (40%) 2-[[2-(4-Chlorphenyl)-4-äthyl -5-oxazolyl]methoxy]-2-methylpropionsäure, Smp. 133,5-134,5° C.

Beispiel 28

Eine Lösung von 0,8 g (5,5 mMol) Methyl-2-hydroxy-2-äthylbutyrat in 2 ml Dimethoxyäthan wurde unter Rühren zu einer Suspension von 0,24 g (6 mMol) einer Dispersion von 60% Natriumhydrid in Mineralöl in 4 ml trockenem Dimethoxyäthan getropft. Die Lösung wurde 10 Minuten bei Raumtemperatur gerührt und dann wurde eine Lösung von 1,21 g (5 mMol) 2-(4-Chlorphenyl)-4-methyl-5-chlormethyloxazol in 5 ml trockenem Dimethoxyäthan zugesetzt. Ein Kristall Natriumjodid wurde zugesetzt und das Gemisch über Nacht unter einer Argonatmosphäre bei Raumtemperatur gerührt. Das Gemisch wurde 1 Stunde bei 65° C erhitzt und nach Abkühlen wurden 2 ml Wasser zugesetzt. Der grösste Teil des Lösungsmittels wurde abgedampft und der Rückstand zwischen 100 ml Diäthyläther und 100 ml Wasser aufgetrennt. Die organische Phase wurde abgetrennt, mit 50 ml Wasser gewaschen und getrocknet. Nach Filtrieren wurde das Filtrat eingedampft und der Rückstand mit 25 ml Aethanol, 0.3 g (7,5 mMol) Natriumhydroxid und 10 ml Wasser vermischt. Die Lösung wurde 40 Minuten am Rückfluss erhitzt, abgekühlt und der grösste Teil des Lösungsmittels abgedampft. Der Rückstand wurde in 100 ml Wasser gelöst und zweimal mit 100 ml Diäthyläther extrahiert. Die wässrige Phase wurde mit konzentrierter Salzsäure auf pH 2 angesäuert und dreimal mit 35 ml Dichlormethan extrahiert. Die vereinigten organischen Extrakte wurden getrocknet, filtriert und eingedampft. Man erhielt nach Umkristallisieren aus Aethylacetat/Hexan 0,6 g (33%) 2-[[2-(4-Chlorphenyl)-4-methyl-5-oxazolyl]methoxy] -2-äthylbuttersäure, Smp. 157° C.

Beispiel 29

Analog Beispiel 1 erhielt man aus 5,5 g (23 mMol) 2-(4-Chlorphenyl)-4-methyl-5-(2-hydroxyäthyl)oxazol (hergestellt wie beschrieben in Acta Pharmaceutica Suecica, 4(S), 1967, 269-280), nach Umkristallisieren aus Aethylacetat/Hexan 0,4 g (5,2%) 2-[2-[2-(4-Chlorphenyl)-4-methyl-5-oxazolyl]äthoxy] -2-methylpropionsäure, Smp. 114-115° C.

Beispiel 30

Analog Beispiel 1 erhielt man unter Verwendung von Aethylmethylketon aus 3,5 g (15,6 mMol) 2-(4-Chlorphenyl)-4-methyl-5-oxazolmethanol nach Umkristallisieren aus Toluol 0,47 g (9,27%) 2-[[2-(4-Chlorphenyl)-4-methyl-5-oxazolyl]methoxy] -2-methylbuttersäure, Smp. 143-144 °C.

Beispiel 31

Analog Beispiel 10 erhielt man aus 5,2 g (21,5 mMol) 2-(4-Chlorphenyl)-5-chlormethyl-4-methyloxazol [aus 2-(4-Chlormethyl) -4-methyl-5-oxazolmethanol hergestellt wie beschrieben im britischen Patent Nr. 1,139,940], 7 g eines Gemisches von Methyl-2-[[2-(4-chlorphenyl)-4-methyl-5-oxazolyl]methoxy] -2-methylpropionat und 2-(4-Chlorphenyl)-5-methoxymethyl-4-methyloxazolin einem Verhältnis von etwa 5:1. Nach Hydrolyse des Gemisches mit einem Ueberschuss an wässrig-äthanolischem Natriumhydroxid erhielt man analog Beispiel 10 4,0 g (60%) 2-[[2-(4-Chlorphenyl)-4-methyl -5-oxazolyl]methoxy]-2-methylpropionsäure, Smp. 166-167 °C das mit dem Produkt von Beispiel 1 identisch war.

In einer Variante wurde das obige Gemisch auf Silicagel mit 30 Vol.% Aethylacetat in Hexan chromatographiert. Nach Abdampfen des Eluats aus den homogenen Fraktionen gefolgt durch Umkristallisieren des Rückstandes aus Hexan erhielt man 5,0 g (72%) Methyl 2-[[2-(4-chlorphenyl)-4-methyl -5-oxazolyl]methoxy]-2-methylpropionat, Smp. 88-90 °C.

Beispiel 32

Aethyl 2-hydroxy-2-methylpropionat wurde mit 2-(4-Chlorphenyl)-5-chlormethyl-4-methyloxazol analog Beispiel 16 umgesetzt. Das Produkt wurde wie beschrieben im Beispiel 31 (2. Absatz) chromatographiert. Man erhielt nach Umkristallisieren aus Hexan, Aethyl-2-[[2-(4-chlorphenyl)-4-methyl -5-oxazolyl]methoxy]-2-methylpropionat, Smp. 71-73 °C das identisch mit dem Produkt von Beispiel 26 war.

Beispiel 33

10,0 ml einer 1M Natriumisopropoxydlösung in Isopropanol wurden einer warmen Lösung von 3,09 g (10 mMol) 2-[[2-(4-Chlorphenyl)-4-methyl-5-oxazolyl]methoxy] -2-methylpropionsäure in 30 ml Isopropanol unter Rühren zugesetzt und das Gemisch wurde abgekühlt. Man erhielt 3,0 g (90%) Natrium-2-[[2-(4-chlorphenyl)-4-methyl-5-oxazolyl]methoxy] -2-methylpropionat, Smp. 277-278 °C.

Die folgenden Beispiele illustrieren pharmazeutische Präparate auf der Basis einer Verbindung der Formel I oder eines pharmazeutisch verwendbaren Salzes davon mit einer Base.

Beispiel A

In an sich bekannter Weise wurden Tabletten folgender Zusammensetzung hergestellt:

| Bestandteil | | pro Tablette |
|---|---|---|
| Wirkstoff | | 50 mg |
| Laktose | | 120 mg |
| Maisstärke | | 75 mg |
| Talk | | 4 mg |
| Magnesiumstearat | | 1 mg |
| | Gesamtgewicht der Tablette | 250 mg |

Beispiel B

In an sich bekannter Weise wurden Kapseln folgender Zusammensetzung hergestellt:

| Bestandteil | pro Kapsel |
|---|---|
| Wirkstoff | 100 mg |
| Laktose | 150 mg |
| Maisstärke | 20 mg |
| Talk | 5 mg |
| Gesamtgewicht für eine Kapsel | 275 mg |

## Patentansprüche

### Patentansprüche für folgende Vertragsstaaten : BE, CH, DE, FR, GB, IT, LI, LU, NL, SE

1. Verbindungen der allgemeinen Formel

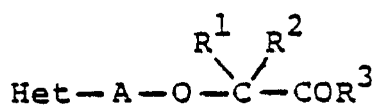

I

worin A $C_{1-6}$-Alkylen,

| | |
|---|---|
| Het | gegebenenfalls heterocyclisch $C_{1-6}$-alkyliertes 2-R-Oxazol-5-yl, 5-R-Oxazol-2-yl, 4-R-Oxazol-2-yl, 2-R-Oxazol-4-yl, 3-R-Isoxazol-5-yl oder 5-R-Isoxazol-3-yl, |
| R | durch Halogen, Trifluormethyl oder $C_{1-6}$-Alkylthio mono- oder disubstituiertes Phenyl oder Thienyl, |
| $R^1$ und $R^2$ | $C_{1-6}$-Alkyl, |
| $R^3$ | Hydroxy, $C_{1-6}$-Alkoxy oder $-NR^4R^5$, |
| $R^4$ und $R^5$ | Wasserstoff oder $C_{1-6}$-Alkyl, oder $-NR^4R^5$ ein Pyrrolodino-, Piperidino-, Morpholino- oder Thiamorpholino-Ring sind, 3-(4-Chlorphenyl)-5-[[1-methyl-1-(piperidinocarbonyl)-äthoxy]methyl]isoxazol |

und pharmazeutisch verwendbare Salze der Säuren der Formel I, worin $R^3$ Hydroxy ist, mit Basen.

2. Verbindungen nach Anspruch 1, worin A Methylen, R durch Halogen oder Trifluormethyl mono- oder disubstituiertes Phenyl, $R^3$ Hydroxy, $C_{1-6}$-Alkoxy oder $-NR^4R^5$, und $R^4$ und $R^5$ Wasserstoff oder $C_{1-6}$-Alkyl sind.

3. Verbindungen nach Anspruch 1, worin A Methylen, 1,1-Aethylen oder 1,2-Aethylen ist.

4. Verbindungen nach Anspruch 1, 2 oder 3, worin Het gegebenenfalls $C_{1-6}$-alkyliertes 2-R-Oxazol-5-yl ist.

5. Verbindungen nach einem der Ansprüche 1-4, worin R Monohalophenyl ist.

6. Verbindungen nach einem der Ansprüche 1-5, worin $R^1$ und $R^2$ Methyl sind.

7. Verbinungen nach einem der Ansprüche 1-6, worin $R^3$ Hydroxy ist.

8. 2-[[2-(4-Chlorphenyl)-4-methyl-5-oxazolyl]methoxy]-2methylpropionsäure.

9. Eine Verbindung nach Anspruch 1, aus der Gruppe der folgenden

   2-[[2-(3,4-Dichlorphenyl)-4-methyl-5-oxazolyl]methoxy]-2-methylpropionsäure,
   2-[[2-(3-Chlorphenyl)-4-methyl-5-oxazolyl]methoxy-2-methylpropionsäure,
   2-Methyl-2-[[4-methyl-2-[4-(methylthio)phenyl]-5-oxazolyl]methoxy]propionsäure,
   2-[[2-(4-Chlorphenyl)-4-methyl-5-oxazolyl]methoxy]-2-methylpropionamid,
   Aethyl 2-[[2-(4-chlorphenyl)-4-methyl-5-oxazolyl]methoxy]-2-methylpropionat,
   2-[[2-(4-Chlorphenyl)-4-äthyl-5-oxazolyl]methoxy]-2-methylpropionsäure,

2-[[2-(4-Chlorphenyl)-4-methyl-5-oxazolyl]methoxy]-2-äthylbuttersäure,
2-[2-[2-(4-Chlorphenyl)-4-methyl-5-oxazolyl]äthoxy]-2-methylpropionsäure,
2-[[2-(4-Chlorphenyl)-4-methyl-5-oxazolyl]methoxy]-2-methylbuttersäure,
Methyl-2-[[2-(4-chlorphenyl)-4-methyl-5-oxazolyl]methoxy]-2-methylpropionat,
2-[2-[3-(4-Chlorphenyl)-5-isoxazolyl]äthoxy]-2-methylpropionsäure,
Methyl 2-[[3-(3,4-dichlorphenyl)-5-isoxazolyl]methoxy]-2-methylpropionat,
2-[[3-(3,4-Dichlorphenyl)-5-isoxazolyl]methoxy]-2-methylpropionsäure,
2-[1-[3-(4-Chlorphenyl)-5-isoxazolyl]äthoxy]-2-methylpropionsäure,
2-[[3-(5-Chlorthien-2-yl)-5-isoxazolyl]methoxy]-2-methylpropionsäure,
2-[[3-(4-Chlorphenyl)-5-isoxazolyl]methoxy]-2-methylpropionamid,
2-[[3-(4-Chlorphenyl)-5-isoxazolyl]methoxy[-N,2-dimethylpropionamid und
3-(4-Chlorphenyl)-5-[[1-methyl-1-(piperidinocarbonyl)-äthoxy]methyl]isoxazol.

**10.** Eine Verbindung nach Anspruch 2, aus der Gruppe der folgenden

2-[[2-(4-Fluorphenyl)-4-methyl-5-oxazolyl]methoxy]-2-methylpropionsäure,
2-[[2-(4-Trifluormethylphenyl)-4-methyl-5-oxazolyl]methoxy]-2-methylpropionsäure,
2-[[2-(4-Chlorphenyl)-5-oxazolyl]methoxy]-2-methylpropionsäure,
2-[[5-(4-Chlorphenyl)-2-oxazolyl]methoxy]-2-methylpropionsäure,
2-[[4-(4-Chlorphenyl)-2-oxazolyl]methoxy]-2-methylpropionsäure,
2-[[2-(4-Trifluormethylphenyl)-5-methyl-4-oxazolyl]methoxy]-2-methylpropionsäure,
2-[[2-(4-Chlorphenyl)-5-methyl-4-oxazolyl]methoxy]-2-methylpropionsäure,
2-[[2-(4-Fluorphenyl)-5-methyl-4-oxazolyl]methoxy]-2-methylpropionsäure,
2-[[2-(4-Chlorphenyl)-4-oxazolyl]methoxy]-2-methylpropionsäure,
2-[[3-(4-Chlorphenyl)-5-isoxazolyl]methoxy]-2-methylpropionsäure,
2-[ [5-(4-Chlorphenyl)-3-isoxazolyl]methoxy]-2-methylpropionsäure und
Methyl 2-[[3-(4-chlorphenyl)-5-isoxazolyl]methoxy]-2-methylpropionat.

**11.** Eine Verbindung nach einem der Ansprüche 1-10 als therapeutisch wirksame Substanz.

**12.** Verfahren zur Herstellung der Verbindungen der Formel I nach Anspruch 1 und der pharmazeutisch verwendbaren Salze dieser Verbindungen, worin $R^3$ Hydroxy ist, mit Basen, dadurch gekennzeichnet, dass man

a) zur Herstellung einer Säure der Formel I, worin $R^3$ Hydroxy ist, einen Alkohol der allgemeinen Formel

Het-A-OH II

mit einem Keton der allgemeinen Formel

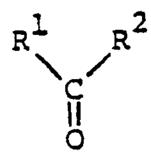

III

worin A, Het, $R^1$ und $R^2$ die im Anspruch 1 angegebene Bedeutung haben,
in Gegenwart eines tri- oder tetrahalogenierten Alkans und einer starken Base umsetzt, oder

b) zur Herstellung einer Verbindung der Formel I, worin $R^3$ $C_{1-6}$-Alkoxy oder $-NR^4R^5$ ist, und $R^4$ und $R^5$
die im Anspruch 1 angegebene Bedeutung haben, eine Verbindung der allgemeinen Formel

Het-A-X IV

mit einer Verbindung der allgemeinen Formel

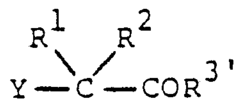

V

worin A, Het, $R^1$ und $R^2$ die im Anspruch 1 angegebene Bedeutung haben, eines von X und Y Hydroxy und das andere ein Abgangsatom oder eine Abgangsgruppe und $R^{3'}$ $C_{1-6}$-Alkoxy oder $-NR^4R^5$ ist, und $R^4$ und $R^5$ die im Anspruch 1 angegebene Bedeutung haben, in Gegenwart einer starken Base umsetzt oder

c) zur Herstellung einer Verbindung der Formel I, worin Het 3-R-Isoxazol-5-yl und $R^3$ $C_{1-6}$-Alkoxy ist, eine Verbindung der allgemeinen Formel

$R-C\equiv N\rightarrow O$ VI

mit einer Verbindung der allgemeinen Formel

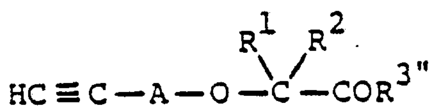

VII

worin R, A, $R^1$ und $R^2$ die im Anspruch 1 angegebene Bedeutung haben und $R^{3''}$ $C_{1-6}$-Alkoxy ist, umsetzt oder

d) zur Herstellung einer Säure der Formel I, worin $R^3$ Hydroxy ist, eine Verbindung der Formel I, worin $R^3$ $C_{1-6}$-Alkoxy ist, hydrolysiert oder

e) zur Herstellung eines Esters der Formel I, worin $R^3$ $C_{1-6}$-Alkoxy ist, eine Verbindung der Formel I, worin $R^3$ Hydroxy ist, verestert oder

f) zur Herstellung eines Amids der Formel I, worin $R^3$ eine Gruppe $-NR^4R^5$ ist, und $R^4$ und $R^5$ die im Anspruch 1 angegebene Bedeutung haben, eine Verbindung der Formel I, worin $R^3$ Hydroxy oder $C_{1-6}$-Alkoxy ist, amidiert und

g) gewünschtenfalls eine Säure der Formel I, worin $R^3$ Hydroxy ist, in ein pharmazeutisch verwendbares Salz mit einer Base umwandelt.

**13.** Pharmazeutisches Präparat auf der Basis einer Verbindung nach einem der Ansprüche 1-10.

**14.** Verwendung einer Verbindung nach einem der Ansprüche 1-10, zur Herstellung eines pharmazeutischen Präparats für die Prophylaxe und Behandlung der Arthritis.

**Patentansprüche für folgenden Vertragsstaat : AT**

**1.** Verfahren zur Herstellung der Verbindungen der allgemeinen Formel

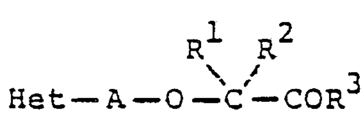

I

worin A $C_{1-6}$-Alkylen,

| | |
|---|---|
| Het | gegebenenfalls heterocyclisch $C_{1-6}$-alkyliertes 2-R-Oxazol-5-yl, 5-R-Oxazol-2-yl, 4-R-Oxazol-2-yl, 2-R-Oxazol-4-yl, 3-R-Isoxazol-5-yl oder 5-R-Isoxazol-3-yl, |
| R | durch Halogen, Trifluormethyl oder $C_{1-6}$-Alkylthio mono- oder disubstituiertes Phenyl oder Thienyl, |
| $R^1$ und $R^2$ | $C_{1-6}$-Alkyl, |
| $R^3$ | Hydroxy, $C_{1-6}$-Alkoxy oder $-NR^4R^5$, |
| $R^4$ und $R^5$ | Wasserstoff oder $C_{1-6}$-Alkyl, oder $-NR^4R^5$ ein Pyrrolidino-, Piperazino-, Morpholino- oder Thiamorpholino-sind, 3-(4-Chlorphenyl)-5-[[1-methyl-1-(piperidinocarbonyl)- |

äthoxy]methyl]isoxazol
und der pharmazeutisch verwendbaren Salze der Säuren der Formel I, worin $R^3$ Hydroxy ist, mit Basen, dadurch gekennzeichnet, dass man

a) zur Herstellung einer Säure der Formel I, worin $R^3$ Hydroxy ist, einen Alkohol der allgemeinen Formel

Het-A-OH II

mit einem Keton der allgemeinen Formel

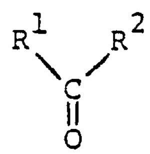

III

worin A, Het, $R^1$ und $R^2$ die im Anspruch 1 angegebene Bedeutung haben,
in Gegenwart eines tri- oder tetrahalogenierten Alkan und einer starken Base umsetzt, oder
b) zur Herstellung einer Verbindung der Formel I worin $R^3$ $C_{1-6}$-Alkoxy oder $-NR^4R^5$ ist, und $R^4$ und $R^5$ die im Anspruch 1 angegebene Bedeutung haben, eine Verbindung der allgemeinen Formel

Het-A-X IV

mit einer Verbindung der allgemeinen Formel

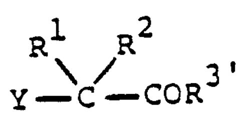

V

worin A, Het, $R^1$ und $R^2$ die im Anspruch 1 angegebene Bedeutung haben, eines von X und Y Hydroxy und das andere ein Abgangsatom oder eine Abgangsgruppe und $R^{3'}$ $C_{1-6}$-Alkoxy oder $-NR^4R^5$ ist, und $R^4$ und $R^5$ die im Anspruch 1 angegebene Bedeutung haben,
in Gegenwart einer starken Base umsetzt oder
c) zur Herstellung einer Verbindung der Formel I, worin Het 3-R-Isoxazol-5-yl und $R^3$ $C_{1-6}$-Alkoxy ist, eine Verbindung der allgemeinen Formel

$R-C\equiv N\rightarrow O$ VI

mit einer Verbindung der allgemeinen Formel

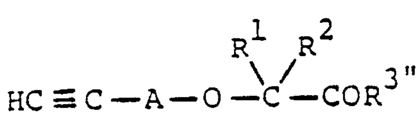

VII

worin R, A, $R^1$ und $R^2$ die im Anspruch 1 angegebene Bedeutung haben und $R^{3''}$ $C_{1-6}$-Alkoxy ist, umsetzt oder
d) zur Herstellung einer Säure der Formel I, worin $R^3$ Hydroxy ist, eine Verbindung der Formel I, worin $R^3$ $C_{1-6}$-Alkoxy ist, hydrolysiert oder
e) zur Herstellung eines Esters der Formel I, worin $R^3$ $C_{1-6}$-Alkoxy ist, eine Verbindung der Formel I, worin $R^3$ Hydroxy ist, verestert oder
f) zur Herstellung eines Amids der Formel I, worin $R^3$ eine Gruppe $-NR^4R^5$ ist, und $R^4$ und $R^5$ die im Anspruch 1 angegebene Bedeutung haben, eine Verbindung der Formel I, worin $R^3$ Hydroxy oder $C_{1-6}$-Alkoxy ist, amidiert und

g) gewünschtenfalls eine Säure der Formel I, worin $R^3$ Hydroxy ist, in ein pharmazeutisch verwendbares Salz mit einer Base umwandelt.

2. Verfahren nach Anspruch 1, worin A Methylen, R durch Halogen oder Trifluormethyl mono- oder disubstituiertes Phenyl und $R^3$ Hydroxy, $C_{1-6}$-Alkoxy oder $-NR^4R^5$, und $R^4$ und $R^5$ Wasserstoff oder $C_{1-6}$-Alkyl sind.

3. Verfahren nach Anspruch 1, worin A Methylen, 1,1-Aethylen oder 1,2-Aethylen ist.

4. Verfahren nach Anspruch 1, 2 oder 3, worin Het gegebenenfalls $C_{1-6}$-alkyliertes 2-R-Oxazol-5-yl ist.

5. Verfahren nach einem der Ansprüche 1-4, worin R Monohalophenyl ist.

6. Verfahren nach einem der Ansprüche 1-5, worin $R^1$ und $R^2$ Methyl sind.

7. Verfahren nach einem der Ansprüche 1-6, worin $R^3$ Hydroxy ist.

8. Verfahren nach einem der Ansprüche 1-6, zur Herstellung der 2-[[2-(4-Chlorphenyl)-4-methyl-5-oxazolyl]methoxy]-2-methylpropionsäure.

9. Verfahren nach Anspruch 1, zur Herstellung einer Verbindung aus der Gruppe der folgenden:

2-[[2-(3,4-Dichlorphenyl)-4-methyl-5-oxazolyl]methoxy]-2-methylpropionsäure,
2-[[2-(3-Chlorphenyl)-4-methyl-5-oxazolyl]methoxy-2-methylpropionsäure,
2-Methyl-2-[[4-methyl-2-[4-(methylthio)phenyl]-5-oxazolyl]methoxy]propionsäure,
2-[[2-(4-Chlorphenyl)-4-methyl-5-oxazolyl]methoxy]-2-methylpropionamid,
Aethyl-2-[[2-(4-chlorphenyl)-4-methyl-5-oxazolyl]methoxy]-2-methylpropionat,
2-[[2-(4-Chlorphenyl)-4-äthyl-5-oxazolyl]methoxy]-2-methylpropionsäure,
2-[[2-(4-Chlorphenyl)-4-methyl-5-oxazolyl]methoxy]-2-äthylbuttersäure,
2-[2-[2-(4-Chlorphenyl)-4-methyl-5-oxazolyl]ethoxy]-2-methylpropionsäure,
2-[[2-(4-Chlorphenyl)-4-methyl-5-oxazolyl]methoxy]-2-methylbuttersäure,
Methyl-2-[[2-(4-chlorphenyl)-4-methyl-5-oxazolyl]methoxy]-2-methylpropionat,
2-[2-[3-(4-Chlorphenyl)-5-isoxazolyl]äthoxy]-2-methylpropionsäure,
Methyl-2-[[3-(3,4-dichlorphenyl)-5-isoxazolyl]methoxy]-2-methylpropionat,
2-[[3-(3,4-Dichlorphenyl)-5-isoxazolyl]methoxy]-2-methylpropionsäure,
2-[1-[3-(4-Chlorphenyl)-5-isoxazolyl]äthoxy]-2-methylpropionsäure,
2-[[3-(5-Chlorthien-2-yl)-5-isoxazolyl]methoxy]-2-methylpropionsäure,
2-[[3-(4-Chlorphenyl)-5-isoxazolyl]methoxy]-2-methylpropionamid,
2-[[3-(4-Chlorphenyl)-5-isoxazolyl]methoxy]-N,2-dimethylpropionamid und
3-(4-Chlorphenyl)-5-[[1-methyl-1-(piperidinocarbonyl)-äthoxy]methyl]isoxazol.

10. Verfahren nach Anspruch 2, zur Herstellung einer Verbindung aus der Gruppe der folgenden:

2-[[2-(4-Fluorphenyl)-4-methyl-5-oxazolyl]methoxy]-2-methylpropionsäure,
2-[[2-(4-Trifluormethylphenyl)-4-methyl-5-oxazolyl]methoxy]-2-methylpropionsäure,
2-[[2-(4-Chlorphenyl)-5-oxazolyl]methoxy]-2-methylpropionsäure,
2-[[5-(4-Chlorphenyl)-2-oxazolyl]methoxy]-2-methylpropionsäure,
2-[[4-(4-Chlorphenyl)-2-oxazolyl]methoxy]-2-methylpropionsäure,
2-[[2-(4-Trifluormethylphenyl)-5-methyl-4-oxazolyl]methoxy]-2-methylpropionsäure,
2-[[2-(4-Chlorphenyl)-5-methyl-4-oxazolyl]methoxy]-2-methylpropionsäure,
2-[[2-(4-Fluorphenyl)-5-methyl-4-oxazolyl]methoxy]-2-methylpropionsäure,
2-[[2-(4-Chlorphenyl)-4-oxazolyl]methoxy]-2-methylpropionsäure,
2-[[3-(4-Chlorphenyl)-5-isoxazolyl]methoxy]-2-methylpropionsäure,
2-[[5-(4-Chlorphenyl)-3-isoxazolyl]methoxy]-2-methylpropionsäure und
Methyl-2-[[3-(4-chlorphenyl)-5-isoxazolyl]methoxy]-2-methylpropionat.

11. Verwendung einer Verbindung nach einem der Ansprüche 1-10, zur Herstellung von pharmazeutischen Präparaten für die Prophylaxe oder Behandlung der Arthritis.

## Claims

**Claims for the following Contracting States : BE, CH, DE, FR, GB, IT, LI, LU, NL, SE**

1. Compounds of the general formula

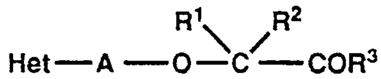

I

wherein A is $C_{1-6}$-alkylene,

| | |
|---|---|
| Het | is optionally heterocyclically $C_{1-6}$-alkylated 2-R-oxazol-5-yl, 5-R-oxazol-2-yl, 4-R-oxazol-2-yl, 2-R-oxazol-4-yl, 3-R-isoxazol-5-yl or 5-R-isoxazol-3-yl, |
| R | is phenyl or thienyl monosubstituted or disubstituted by halogen, trifluoromethyl or $C_{1-6}$-alkylthio, |
| $R^1$ and $R^2$ | are $C_{1-6}$-alkyl, |
| $R^3$ | is hydroxy, $C_{1-6}$-alkoxy or $-NR^4R^5$, |
| $R^4$ and $R^5$ | are hydrogen or $C_{1-6}$-alkyl or $-NR^4R^5$ is pyrrolidino, piperazino, morpholino or thiamorpholino, |

3-(4-chlorophenyl)-5[[1-methyl-1-(piperidinocarbonyl)ethoxy]methyl]isoxazole, and pharmaceutically usable salts of the acids of formula in which $R^3$ is hydroxy with bases.

2. Compounds according to claim 1, wherein A is methylene, R is phenyl mono- or disubstituted by halogen or trifluoromethyl, R is hydroxy, $C_{1-6}$-alkoxy or $-NR^4R^5$ and $R^4$ and $R^5$ are hydrogen or $C_{1-6}$-alkyl.

3. Compounds according to claim 1, wherein A is methylene, 1,1-ethylene or 1,2-ethylene.

4. Compounds according to claim 1, 2 or 3, wherein Het is optionally $C_{1-6}$-alkylated 2-R-oxazol-5-yl.

5. Compounds according to any one of claims 1-4, wherein R is monohalophenyl.

6. Compounds according to any one of claims 1-5, wherein $R^1$ and $R^2$ are methyl.

7. Compounds according to any one of claims 1-6, wherein $R^3$ is hydroxy.

8. 2-[[2-(4-Chlorophenyl)-4-methyl-5-oxazoly]methoxy]-2-methylpropionic acid.

9. A compound according to claim 1 from the following group

   2-[[2-(3,4-dichlorophenyl)-4-methyl-5-oxazolyl]methoxy]-2-methylpropionic acid,
   2-[[2-(3-chlorophenyl)-4-methyl-5-oxazolyl]methoxy]-2-methylpropionic acid,
   2-methyl-2-[[4-methyl-2-[4-(methylthio)phenyl]-5-oxazolyl]methoxy]propionic acid,
   2-[[2-(4-chlorophenyl)-4-methyl-5-oxazolyl]methoxy]-2-methylpropionamide,
   ethyl 2-[[2-(4-chlorophenyl)-4-methyl-5-oxazolyl]-methoxy]-2-methylpropionate,
   2-[[2-(4-chlorophenyl)-4-ethyl-5-oxazolyl]methoxy]-2-methylpropionic acid,
   2-[[2-(4-chlorophenyl)-4-ethyl-5-oxazolyl]methoxy]-2-ethylbutyric acid,
   2-[2-[2-(4-chlorophenyl)-4-methyl-5-oxazolyl]ethoxy]-2-methylpropionic acid,
   2-[[2-(4-chlorophenyl)-4-ethyl-5-oxazolyl]methoxy]-2-methylbutyric acid,
   methyl 2-[[2-(4-chlorophenyl)-4-methyl-5-oxazolyl]methoxy]-2-methylpropionate,
   2-[2-[3-(4-chlorophenyl)-5-isoxazolyl]ethoxy]-2-methylpropionic acid,
   methyl 2-[[3-(3,4-dichlorophenyl)-5-isoxazolyl]methoxy]-2-methylpropionate,
   2-[[3-(3,4-dichlorophenyl)-5-isoxazolyl]methoxy]-2-methylpropionic acid,
   2-[1-[3-(4-chlorophenyl)-5-isoxazolyl]ethoxy]-2-ethylpropionic acid,
   2-[[3-(5-chlorothien-2-yl)-5-isoxazolyl]methoxy]-2-methylpropionic acid,
   2-[[3-(4-chlorophenyl)-5-isoxazolyl]methoxy]-2-methyl-propionamide,
   2-[[3-(4-chlorophenyl)-5-isoxazolyl]methoxy]-N,2-dimethylpropionamide and
   3-(4-chlorophenyl)-5-[[1-methyl-1-(piperidinocarbonyl)-ethoxy]methyl]isoxazole.

**10.** A compound according to claim 2 from the following group

2-[[2-(4-fluorophenyl)-4-methyl-5-oxazolyl]methoxy]-2-methylpropionic acid,
2-[[2-(4-trifluoromethylphenyl)-4-methyl-5-oxazolyl]-methoxy]-2-methylpropionic acid,
2-[[2-(4-chlorophenyl)-5-oxazolyl]methoxy]-2-methyl-propionic acid,
2-[[5-(4-chlorophenyl)-2-oxazolyl]methoxy]-2-methyl-propionic acid,
2-[[4-(4-chlorophenyl)-2-oxazolyl]methoxy]-2-methyl-propionic acid
2-[[2-(4-trifluoromethylphenyl)-5-methyl-4-oxazolyl]-methoxy]-2-methylpropionic acid,
2[[2-(4-chlorophenyl)-5-methyl-4-oxazolyl]methoxy]-2-methylpropionic acid,
2-[[2-(4-fluorophenyl)-5-methyl-4-oxazolyl]methoxy]-2-methylpropionic acid,
2-[[2-(4-chlorophenyl)-4-oxazolyl]methoxy]-2-methyl-propionic acid,
2-[[3-(4-Chlorophenyl)-5-isoxazolyl]methoxy]-2-methyl-propionic acid,
2-[[5-(4-chlorophenyl)-3-isoxazolyl]methoxy]-2-methy-propionic acid and
methyl 2-[[3-(4-chlorophenyl)-5-isoxazolyl]methoxy]-2-methylpropionate.

**11.** A compound according to any one of claim 1-10 for use as a therapeutically active substance.

**12.** A process for the manufacture of the compounds of formula 1 according to claim 1 and the pharmaceutically acceptable salts of those compounds in which $R^3$ is hydroxy with bases, characterized by

a) for the manufacture of an acid if formula I in which $R^3$ is hydroxy, reacting an alcohol of the general formula

Het-A-OH II

within a ketone of the general formula

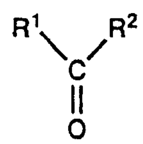

III

wherein A, Het, $R^1$ and $R^2$ have the significance given in claim 1,
in the presence of a tri- or tetrahalogenated alkane and a strong base, or
b) for the manufacture of a compound of formula I in which $R^3$ is $C_{1-6}$-alkoxy or $-NR^4R^5$ and $R^4$ and $R^5$ have the significance given in claim 1, reacting a compound of the general formula

Het-A-X IV

with a compound of the general formula

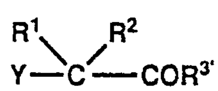

V

wherein A, Het, $R^1$ and $R^2$ have the significance given in claim 1, one of X and Y is hydroxy and the other is a leaving atom or a leaving group and $R^{3'}$ is $C_{1-6}$-alkoxy or $-NR^4R^5$ and $R^4$ and $R^5$ have the significance given in claim 1,
in the presence of a strong base, or
c) for the manufacture of a compound of formula I in which Het is 3-R-isoxazol-5-yl and $R^3$ is $C_{1-6}$-alkoxy, reacting a compound of the general formula

$R\text{-}C\equiv N\rightarrow O$ VI

with a compound of the general formula

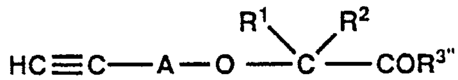 VII

wherein R, A, $R^1$ and $R^2$ have the significance given in claim 1 and $R^{3''}$ is $C_{1-6}$-alkoxy, or

d) for the manufacture of an acid of formula I in which $R^3$ is hydroxy, hydrolyzing a compound of formula I in which $R^3$ is $C_{1-6}$-alkoxy, or

e) for the manufacture of an ester of formula I in which $R^3$ is $C_{1-6}$-alkoxy, esterifying a compound of formula I in which $R^3$ is hydroxy, or

f) for the manufacture of an amide of formula I in which $R^3$ is a group $-NR^4R^5$ and $R^4$ and $R^5$ have the significance given in claim 1, amidating a compound of formula I in which $R^3$ is hydroxy or $C_{1-6}$-alkoxy, and

g) if desired, converting an acid of formula I in which $R^3$ is hydroxy into a pharmaceutically usable salt with a base.

**13.** A pharmaceutical preparation based on a compound according to any one claims 1-10.

**14.** The use of a compound according to any one of claims 1-10 for the manufacture of a pharmaceutical preparation for the prophylaxis and treatment of arthritis.

**Claims for the following Contracting State : AT**

**1.** Process for the manufacture of compounds of the general formula

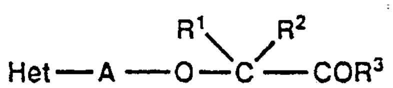 I

wherein A is $C_{1-6}$-alkylene,

| | |
|---|---|
| Het | is optionally heterocyclically $C_{1-6}$-alkylated 2-R-oxazol-5-yl, 5-R-oxazol-2-yl, 4-R-oxazol-2-yl, 2-R-oxazol-4-yl, 3-R-isoxazol-5-yl or 5-R-isoxazol-3-yl, |
| R | is phenyl or thienyl monosubstituted or disubstituted by halogen, trifluoromethyl or $C_{1-6}$-alkylthio, |
| $R^1$ and $R^2$ | are $C_{1-6}$-alkyl, |
| $R^3$ | is hydroxy, $C_{1-6}$-alkoxy or $-NR^4R^5$, |
| $R^4$ and $R^5$ | are hydrogen or $C_{1-6}$-alkyl or $-NR^4R^5$ is pyrrolidino, piperazino, morpholino or thiamorpholino, |

3-(4-chlorophenyl)-5-[[1-methyl-1-(piperidinocarbonyl)ethoxy]-methyl]isoxazole, and of pharmaceutically usable salts of the acids of formula I in which $R^3$ is hydroxy with bases characterized by,

a) for the manufacture of an acid if formula I in which $R^3$ is hydroxy, reacting an alcohol of the general formula

Het-A-OH II

within a ketone of the general formula

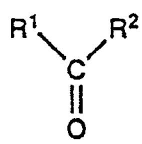 III

wherein A, Het, $R^1$ and $R^2$ have the significance given in claim 1,
in the presence of a tri- or tetrahalogenated alkane and a strong base, or

b) for the manufacture of a compound of formula I in which $R^3$ is $C_{1-6}$-alkoxy or $-NR^4R^5$ and $R^4$ and $R^5$ have the significance given in claim 1, reacting a compound of the general formula

Het-A-X IV

with a compound of the general formula

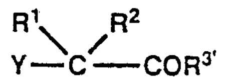

V

wherein A, Het, $R^1$ and $R^2$ have the significance given in claim 1, one of X and Y is hydroxy and the other is a leaving atom or a leaving group and $R^{3'}$ is $C_{1-6}$-alkoxy or $-NR^4R^5$ and $R^4$ and $R^5$ have the significance given in claim 1,
in the presence of a strong base, or
c) for the manufacture of a compound of formula I in which Het is 3-R-isoxazol-5-yl and $R^3$ is $C_{1-6}$-alkoxy, reacting a compound of the general formula

$R\text{-}C{\equiv}N{\rightarrow}O$ VI

with a compound of the general formula

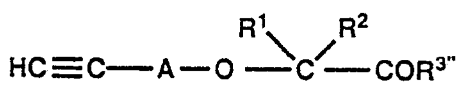

VII

wherein R, A, $R^1$ and $R^2$ have the significance given in claim 1 and $R^{3''}$ is $C_{1-6}$-alkoxy,
or
d) for the manufacture of an acid of formula I in which $R^3$ is hydroxy, hydrolyzing a compound of formula I in which $R^3$ is $C_{1-6}$-alkoxy, or
e) for the manufacture of an ester of formula I in which $R^3$ is $C_{1-6}$-alkoxy, esterifying a compound of formula I in which $R^3$ is hydroxy, or
f) for the manufacture of an amide of formula 1 in which $R^3$ is a group $-NR^4R^5$ and $R^4$ and $R^5$ have the significance given in claim 1, amidating a compound of formula 1 in which $R^3$ is hydroxy or $C_{1-6}$-alkoxy, and
g) if desired, converting an acid of formula 1 in which $R^3$ is hydroxy into a pharmaceutically usable salt with a base.

2. A process according to claim 1, wherein A is methylene, R is phenyl mono- or disubstituted by halogen or trifluoromethyl, $R^3$ is hydroxy, $C_{1-6}$-alkoxy or $-NR^4R^5$ and $R^4$ and $R^5$ are hydrogen or $C_{1-6}$-alkyl.

3. A process according to claim 1, wherein A is methylene, 1,1-ethylene or 1,2-ethylene.

4. A process according to claim 1, 2 or 3, wherein Het is optionally $C_{1-6}$-alkylated 2-R-oxazol-5-yl.

5. A process according to any one of claims 1-4, wherein R is monohalophenyl.

6. A process according to any one of claims 1-5, wherein $R^1$ and $R^2$ are methyl.

7. A process according to any one of claims 1-6, wherein $R^3$ is hydroxy.

8. A process according to any one of claims 1-6 for the manufacture of 2-[[2-(4-chlorophenyl)-4-methyl-5-oxazoly]methoxy]-2-methylpropionic acid.

9. A process according to claim 1 from the manufacture of

2-[[2-(3,4-dichlorophenyl)4-methyl-5-oxazolyl]methoxy]-2-methylpropionic acid,
2-[[2-(3-chlorophenyl)-4-methyl-5-oxazolyl]methoxy]-2-methylpropionic acid,
2-methyl-2-[[4-methyl-2-[4-(methylthio)phenyl]-5-oxazolyl]methoxy]propionic acid,
2-[[2-(4-chlorophenyl)-4-methyl-5-oxazolyl]methoxy]-2-methylpropionamide,

ethyl 2-[[2-(4-chlorophenyl)-4-methyl-5-oxazolyl]methoxy]-2-methylpropionate,
2-[[2-(4-chlorophenyl)-4-ethyl-5-oxazolyl]methoxy]-2-methylpropionic acid,
2-[[2-(4-chlorophenyl)-4-ethyl-5-oxazolyl]methoxy]-2-ethylbutyric acid,
2-[2-[2-(4-chlorophenyl)-4-methyl-5-oxazolyl]ethoxy]-2-methylpropionic acid,
2-[[2-(4-chlorophenyl)-4-ethyl-5-oxazolyl]methoxy]-2-methylbutyric acid,
methyl 2-[[2-(4-chlorophenyl)-4-methyl-5-oxazolyl]-methoxy]-2-methylpropionate,
2-[2-[3-(4-chlorophenyl)-5-isoxazolyl]ethoxy]-2-methylpropionic acid,
methyl 2-[[3-(3,4-dichlorophenyl)-5-isoxazolyl]methoxy]-2-methylpropionate,
2-[[3-(3,4-dichlorophenyl)-5-isoxazolyl]methoxy]-2-methylpropionic acid,
2-[1-[3-(4-chlorophenyl)-5-isoxasolyl]ethoxy]-2-ethylpropionic acid,
2-[[3-(5-chlorothien-2-yl)-5-isoxazolyl]methoxy]-2-methylpropionic acid,
2-[[3-(4-chlorophenyl)-5-isoxazolyl]methoxy]-2-methylpropionamide,
2-[[3-(4-chlorophenyl)-5-isoxazolyl]methoxy]-N,2-dimethylpropionamide and
3-(4-chlorophenyl)-5-[[1-methyl-1-(piperidinocarbonyl)-ethoxy]methyl]isoxazole.

**10.** A process according to claim 2 for the manufacture of

2-[[2-(4-fluorophenyl)-4-methyl-5-oxazolyl]methoxy]-2-methylpropionic acid,
2-[[2-(4-trifluoromethylphenyl)-4-methyl-5-oxazolyl]methoxy]-2-methylpropionic acid,
2-[[2-(4-chlorophenyl)-5-oxazolyl]methoxy]-2-methylpropionic acid,
2-[[5-(4-chlorophenyl)-2-oxazolyl]methoxy]-2-methylpropionic acid,
2-[[4-(4-chlorophenyl)-2-oxazolyl]methoxy]-2-methylpropionic acid
2-[[2-(4-trifluoromethylphenyl)-5-methyl-4-oxazolyl]methoxy]-2-methylpropionic acid,
2[[2-(4-chlorophenyl)-5-methyl-4-oxazolyl]methoxy]-2-methylpropionic acid,
2-[[2-(4-fluorophenyl)-5-methyl-4-oxazolyl]methoxy]-2-methylpropionic acid,
2-[[2-(4-chlorophenyl)-4-oxazolyl]methoxy]-2-methylpropionic acid,
2-[[3-(4-Chlorophenyl)-5-isoxazolyl]methoxy]-2-methylpropionic acid,
2-[[5-(4-chlorophenyl)-3-isoxazolyl]methoxy]-2-methypropionicacid and
methyl 2-[[3-(4-chlorophenyl)-5-isoxazolyl]methoxy]-2-methylpropionate.

**11.** The use of a compound according to any one of claims 1-10 for the manufacture of a pharmaceutical preparation for the prophylaxis and treatment of arthritis.

**Revendications**

**Revendications pour les Etats contractants suivants : BE, CH, DE, FR, GB, IT, LI, LU, NL, SE**

**1.** Composés de formule générale :

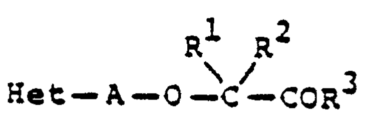

I

où

| | |
|---|---|
| A | représente un alcoylène en $C_{1-6}$, |
| Het | un radical 2-R-oxazol-5-yle, 5-R-oxazol-2-yle, 4-R-oxazol-2-yle, 2-R-oxazol-4-yle, 3-R-isoxazol-5-yle, ou 5-R-isoxazol-3-yle éventuellement alcoylé de façon hétérocyclique avec un alcoyle en $C_{1-6}$, |
| R | représente un phényle ou thiényle mono-ou disubstitué par un halogène, trifluorométhyle ou alcoylthio en $C_{1-6}$ |
| $R^1$ et $R^2$ | représentent un alcoyle en $C_{1-6}$, |
| $R^3$ | représente un hydroxy, alcoxy en $C_{1-6}$ ou $-NR^4R^5$, |
| $R^4$ et $R^5$ | représentent un hydrogène ou un alcoyle en $C_{1-6}$, ou $-NR^4R^5$ est un noyau pyrrolidinyle, pipérazinyle, morpholinyle ou thiomorpholinyle, 3-(4-chlorophényl)-5-[[1-méthyl-1-(pipéridinocarbonyl)-éthoxy]méthyl]isoxazole, |

et les sels pharmaceutiquement acceptables des acides de formule I, où $R^3$ est un hydroxy, avec des bases.

2. Composés selon la revendication 1, où A représente un méthylène, R représente un phényle mono- ou disubstitué par un halogène ou un trifluorométhyle, $R^3$ un hydroxy, alcoxy en $C_{1-6}$ ou $-NR^4R^5$, et $R^4$ et $R^5$ représentent un hydrogène ou un alcoyle en $C_{1-6}$;

3. Composés selon la revendication 1, où A représente un méthylène, 1,1-éthylène ou 1,2-éthylène.

4. Composés selon les revendications 1, 2 ou 3, où Het représente un 2-R-oxazol-5-yle éventuellement alcoylé par un alcoyle en $C_{1-6}$.

5. Composés selon l'une des revendications 1-4, où R est un monohalophényle.

6. Composés selon l'une des revendications 1-5, où $R^1$ et $R^2$ représentent un méthyle.

7. Composés selon l'une des revendications 1-6, où $R^3$ est un hydroxy.

8. Acide 2-[[2-(4-chlorophényl)-4-méthyl-5-oxazolyl]méthoxy]-2-méthylpropionique.

9. Composé selon la revendication 1, du groupe des composés suivants
Acide 2-[[2-(3,4-dichlorophényl)-4-méthyl-5-oxazolyl]méthoxy]-2-méthylpropionique,
Acide 2-[[2-(3-chlorophényl)-4-méthyl-5-oxazolyl]méthoxy-2-méthylpropionique,
Acide 2-méthyl-2-[[4-méthyl-2-[4-(méthylthio)phényl]-5-oxazolyl]méthoxy)propionique,
2-[[2-(4-chlorophényl)-4-méthyl-5-oxazolyl]méthoxy]-2-méthylpropionamide,
2-[[2-(4-chlorophényl)-4-méthyl-5-oxazolyl]méthoxy]-2-méthylpropionate d'éthyle,
Acide 2-[[2-(4-chlorophényl)-4-éthyl-5-oxazolyl]méthoxy]-2-méthylpropionique,
Acide 2-[[2-(4-chlorophényl)-4-méthyl-5-oxazolyl]méthoxy]-2-éthyl-butyrique,
Acide 2-[2-[2-(4-chlorophényl)-4-méthyl-5-oxazolyl]éthoxy]-2-méthylpropionique,
Acide 2-[[2-(4-chlorophényl)-4-méthyl-5-oxazolyl]méthoxyl-2-méthylbutyrique,
2-[[2-(4-chlorophényl)-4-méthyl-5-oxazolyl]méthoxy]-2-méthylpropionate de méthyle,
Acide 2-[2-[3-(4-chlorophényl)-5-isoxazolyl]éthoxy]-2-méthyl-propionique,
2-[[3-(3,4-dichlorophényl)-5-isoxazolyl]méthoxy]-2-méthylpropionate de méthyle,
Acide 2-[[3-(3,4-dichlorophényl)-5-isoxazolyl]méthoxy]-2-méthylpropionique,
Acide 2-[1-[3-(4-chlorophényl)-5-isoxazolyl]éthoxy]-2-méthyl-propionique,
Acide 2-[[3-(5-chlorothién-2-yl)-5-isoxazolyl]méthoxy]-2-méthylpropionique,
2-[[3-(4-chlorophényl)-5-isoxazolyl]méthoxy]-2-méthyl-propionamide,
2-[[3-(4-chlorophényl)-5-isoxazolyl]méthoxy]-N,2-diméthylpropionamide et
3-(4-chlorophényl -5-[[1-méthyl-1-(pipéridinocarbonyl)-éthoxy)méthyl]isoxazole.

10. Composé selon la revendication 2, du groupe des composés suivants :
Acide 2-[[2-(4-fluorophényl)-4-méthyl-5-oxazolyl]méthoxy]-2-méthylpropionique,
Acide 2-[[2-(4-trilfuorométhylphényl)-4-méthyl-5-oxazolyl]méthoxy]-2-méthylpropionique,
Acide 2-[[2-(4-chlorophényl)-5-oxazolyl]méthoxy)-2-méthyl-propionique,
Acide 2-[[5-(4-chlorophényl)-2-oxazolyl] méthoxy]-2-méthyl-propionique,
Acide 2-[[4-(4-chlorophényl)-2-oxazolyl]méthoxy]-2-méthylpropionique,
Acide 2-[[2-(4-trifluorométhylphényl)-5-méthyl-4-oxazolyl]méthoxy]-2-méthylpropionique,
Acide 2-[[2-(4-chlorophényl)-5-méthyl-4-oxazolyl]méthoxy]-2-méthylpropionique,
Acide 2-[[2-(4-fluorophényl)-5-méthyl-4-oxazolyl]méthoxy]-2-méthylpropionique,
Acide 2-[[2-(4-chlorophényl)-4-oxazolyl]méthoxy)-2-méthylpropionique,
Acide 2-[[3-(4-chlorophényl)-5-isoxazolyl]méthoxy]-2-méthylpropionique,
Acide 2-[[5-(4-chlorophényl)-3-isoxazolyl]méthoxy]-2-méthylpropionique, et
2-[[3-(4-chlorophényl)-5-isoxazolyl]méthoxy]-2-méthylpropionate de méthyle.

11. Composé selon l'une des revendications 1-10 comme substance thérapeutiquement active.

12. Procédé de préparation des composés de formule I selon la revendication 1 et des sels pharmaceutiquement acceptables de ces composés, où $R^3$ est un hydroxy, avec des bases,
caractérisé en ce que
a) Pour préparer un acide de formule I, où $R^3$ est un hydroxy, on fait réagir un alcool de formule générale

Het-A-OH II

avec une cétone de formule générale

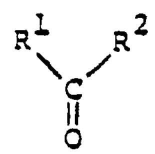

III

où A, Het, $R^1$ et $R^2$ ont la signification donnée dans la revendication 1,
en présence d'un alcane tri- ou tétrahalogéné et d'une base forte, ou
b) pour préparer un composé de formule I où $R^3$ est un alcoxy en $C_{1-6}$ ou $-NR^4R^5$, et $R^4$ et $R^5$ ont la signification donnée dans la revendication 1, on fait réagir un composé de formule générale

Het-A-X IV

avec un composé de formule générale

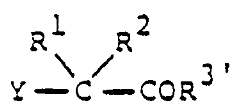

V

où A, Het, $R^1$ et $R^2$ ont la signification donnée dans la revendication 1, l'un des radicaux X et Y représente un hydroxy et l'autre un atome sortant ou un groupe sortant et $R^{3'}$ est un alcoxy en $C_{1-6}$ ou $-NR^4R^5$, et $R^4$ et $R^5$ ont la signification donnée dans la revendication 1,
en présence d'une base forte, ou
c) pour préparer un composé de formule I, où Het est un 3-R-isoxazol-5-yle et $R^3$ est un alcoxy en $C_{1-6}$, on fait réagir un composé de formule générale

$R-C\equiv N\rightarrow O$ VI

avec un composé de formule générale

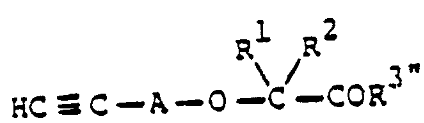

VII

où R, A, $R^1$ et $R^2$ ont la signification donnée dans la revendication 1 et $R^{3''}$ est un alcoxy en $C_{1-6}$, ou
d) pour préparer un acide de formule I, où $R^3$ est un hydroxy, on hydrolyse un composé de formule I ou $R^3$ est un alcoxy en $C_{1-6}$, ou
e) pour préparer un ester de formule I où $R^3$ est un alcoxy en $C_{1-6}$, on estérifie un composé de formule I, où $R^3$ est un hydroxy, ou
f) pour préparer un amide de formule I où $R^3$ est un groupe $-NR^4R^5$, et $R^4$ et $R^5$ ont la signification donnée dans la revendication 1, on amide un composé de formule I où $R^3$ est un hydroxy ou un alcoxy en $C_{1-6}$, et
g) si on le désire, on transforme un acide de formule I, où $R^3$ est un hydroxy en un sel pharmaceutiquement acceptable avec une base.

**13.** Préparation pharmaceutique à base d'un composé selon l'une des revendications 1-10.

**14.** Application d'un composé selon l'une quelconque des revendications 1-10 à la préparation d'une préparation pharmaceutique pour la prophylaxie et le traitement de l'arthrite.

**Revendications pour l'Etat contractant suivant : AT**

**1.** Procédé de préparation des composés de formule générale :

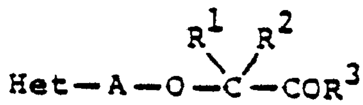

I

où

A représente un alcoylène en $C_{1-6}$,

Het un radical 2-R-oxazol-5-yle, 5-R-oxazol-2-yle, 4-R-oxazol-2-yle, 2-R-oxazol-4-yle, 3-R-isoxazol-5-yle, ou 5-R-isoxazol-3-yle éventuellement alcoylé de façon hétérocyclique avec un alcoyle en $C_{1-6}$,

R représente un phényle ou thiényle mono-ou disubstitué par un halogène, trifluorométhyle ou alcoylthio en $C_{1-6}$

$R^1$ et $R^2$ représentent un alcoyle en $C_{1-6}$,

$R^3$ représente un hydroxy, alcoxy en $C_{1-6}$ ou $-NR^4R^5$,

$R^4$ et $R^5$ représentent un hydrogène ou un alcoyle en $C_{1-6}$, ou $-NR^4R^5$ est un noyau pyrrolidinyle, pipérazinyle, morpholinyle ou thiomorpholinyle, 3-(4-chlorophényl)-5-[[méthyl-1-(pipériinocaronyl)-éthoxy]méthyl]isoxazole,

et les sels pharmaceutiquement acceptables des acides de formule I, où $R^3$ est un hydroxy, avec des bases,

caractérisé en ce que

a) Pour préparer un acide de formule I, où $R^3$ est un hydroxy, on fait réagir un alcool de formule générale

Het-A-OH II

avec une cétone de formule générale

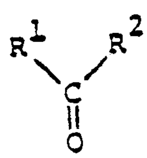

III

où A, Het, $R^1$ et $R^2$ ont la signification donnée dans la revendication 1,
en présence d'un alcane tri- ou tétrahalogéné et d'une base forte, ou

b) pour préparer un composé de formule I où $R^3$ est un alcoxy en $C_{1-6}$ ou $-NR^4R^5$, et $R^4$ et $R^5$ ont la signification donnée dans la revendication 1, on fait réagir un composé de formule générale

Het-A-X IV

avec un composé de formule générale

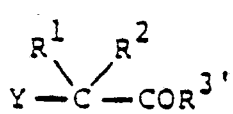

V

où A, Het, $R^1$ et $R^2$ ont la signification donnée dans la revendication 1, l'un des radicaux X et Y

représente un hydroxy et l'autre un atome sortant ou un groupe sortant et $R^{3'}$ est un alcoxy en $C_{1-6}$ ou $-NR^4R^5$, et $R^4$ et $R^5$ ont la signification donnée dans la revendication 1,
en présence d'une base forte, ou
c) pour préparer un composé de formule I, où Het est un 3-R-isoxazol-5-yle et $R^3$ est un alcoxy en $C_{1-6}$, on fait réagir un composé de formule générale

$R-C{\equiv}N{\rightarrow}O$ VI

avec un composé de formule générale

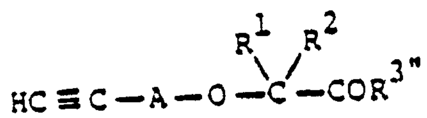

VII

où R, A, $R^1$ et $R^2$ ont la signification donnée dans la revendication 1 et $R^{3''}$ est un alcoxy en $C_{1-6}$, ou
d) pour préparer un acide de formule I, où $R^3$ est un hydroxy, on hydrolyse un composé de formule I où $R^3$ est un alcoxy en $C_{1-6}$, ou
e) pour préparer un ester de formule I où $R^3$ est un alcoxy en $C_{1-6}$, on estérifie un composé de formule I, ou $R^3$ est un hydroxy, ou
f) pour préparer un amide de formule I où $R^3$ est un groupe $-NR^4R^5$, et $R^4$ et $R^5$ ont la signification donnée dans la revendication 1, on amide un composé de formule I ou $R^3$ est un hydroxy ou un alcoxy en $C_{1-6}$, et
g) si on le désire, on transforme un acide de formule I, où $R^3$ est un hydroxy en un sel pharmaceutiquement acceptable avec une base.

2. Procédé selon la revendication 1, dans lequel A représente un méthylène, R un phényle mono- ou disubstitué par un halogène ou un trifluorométhyle et $R^3$ un hydroxy, alcoxy en $C_{1-6}$ ou $-NR^4R^5$, et $R^4$ et $R^5$ représentent un hydrogène ou un alcoyle en $C_{1-6}$.

3. Procédé selon la revendication 1, dans lequel A représente un méthylène, 1,1-éthylène ou 1,2-éthylène.

4. Procédé selon la revendication 1, 2 ou 3, où Het est un 2(R(oxazol-5-yle éventuellement alcoylé par un alcoyle en $C_{1-6}$.

5. Procédé selon l'une des revendications 1-4, où R est un monohalophényle.

6. Procédé selon l'une des revendications 1-5, où $R^1$ et $R^2$ représentent un méthyle.

7. Procédé selon l'une des revendications 1-6, où $R^3$ est un hydroxy.

8. Procédé selon l'une des revendications 1-6, de préparation de l'aciee 2-[[2-(4-chlorophényl)-4-méthyl-5-oxazolyl]méthoxy]-2-méthylpropionique.

9. Procédé selon la revendication 1 de préparation d'un composé du groupe des composés suivants :

Acide 2-[[2-(3,4-dichlorophényl)-4-méthyl-5-oxazolyl]méthoxy]-2-méthylpropionique,
Acide 2-[[2-(3-chlorophényl)-4-méthyl-5-oxazolyl]méthoxy-2-méthylpropionique,
Acide 2-méthyl-2-[[4-méthyl-2-[4-(méthylthio)phényl]-5-oxazolyl]méthoxy]propionique,
2-[[2-(4-chlorophényl)-4-méthyl-5-oxazolyl]méthoxy]-2-méthylpropionamide,
2-[[2-(4-chlorophényl)-4-méthyl-5-oxazolyl]méthoxy]-2-méthylpropionate d'éthyle,
Acide 2-[[2-(4-chlorophényl)-4-éthyl-5-oxazolyl]méthoxy]-2-méthylpropionique,
Acide 2-[[2-(4-chlorophényl)-4-méthyl-5-oxazolyl]méthoxy]-2-éthyl-butyrique,
Acide 2-[2-[2-(4-chlorophényl)-4-méthyl-5-oxazolyl]éthoxy]-2-méthylpropionique,
Acide 2-[[2-(4-chlorophényl)-4-méthyl-5-oxazolyl]méthoxyl-2-méthylbutyrique,
2-[[2-(4-chlorophényl)-4-méthyl-5-oxazolyl]méthoxy]-2-méthylpropionate de méthyle,

Acide 2-[2-[3-(4-chlorophényl)-5-isoxazolyl]éthoxy]-2-méthyl-propionique,
2-[[3-(3,4-dichlorophényl)-5-isoxazolyl]-méthoxy]-2-méthylpropionate de méthyle,
Acide 2-[[3-(3,4-dichlorophényl)-5-isoxazolyl]méthoxy]-2-méthylpropionique,
Acide 2-[1-[3-(4-chlorophényl)-5-isoxazolyl]-éthoxy]-2-méthyl-propionique,
Acide 2-[[3-(5-chlorothién-2-yl)-5-isoxazolyl]méthoxy]-2-méthylpropionique,
2-[[3-(4-chlorophényl)-5-isoxazolyl]méthoxy]-2-méthyl-propionamide,
2-[[3-(4-chlorophényl)-5-isoxazolyl]méthoxy]-N,2-diméthylpropionamide et
3-(4-chlorophényl -5-[[1-méthyl-1-(pipéridinocarbonyl)-éthoxy]méthyl]isoxazole.

**10.** Procédé selon la revendication 2, de préparation d'un composé du groupe des composés suivants :

Acide 2-[[2(4-fluorophényl)-4-méthyl-5-oxazolyl]méthoxy]-2-méthylpropionique,
Acide 2-[[2-(4-trilfuorométhylphényl)-4-méthyl-5-oxazolyl]méthoxy]-2-méthylpropionique,
Acide 2-[[2-(4-chlorophényl)-5-oxazolyl]méthoxy]-2-méthyl-propionique,
Acide 2-[[5-(4-chlorophényl)-2-oxazolyl] méthoxy]-2-méthyl-propionique,
Acide 2-[[4-chlorophényl)-2-oxazolyl]méthoxy]-2-méthylpropionique,
Acide 2-[[2-(4-trifluorométhylphényl)-5-méthyl-4-oxazolyl]méthoxy]-2-méthylpropionique,
Acide 2-[[2-(4-chlorophényl)-5-méthyl-4-oxazolyl]méthoxy]-2-méthylpropionique,
Acide 2-[[2-(4-fluorophényl)-5-méthyl-4-oxazolyl]méthoxy]-2-méthylpropionique,
Acide 2-[[2-(4-chlorophényl)-4-oxazolyl]méthoxy]-2-méthylpropionique,
Acide 2-[[3-(4-chlorophényl)-5-isoxazolyl]méthoxy]-2-méthylpropionique,
Acide 2-[[5-(4-chlorophényl)-3-isoxazolyl]-méthoxy]-2-méthylpropionique, et
2-[[3-(4-chlorophényl)-5-isoxazolyl]méthoxy]-2-méthylpropionate de méthyle.

**11.** Application d'un composé selon l'une des revendications 1-10 à la préparation de préparations pharmaceutiques pour la prophylaxie ou le traitement de l'arthrite.